# EUROPEAN PATENT APPLICATION

(11) **EP 4 825 873 A1**
(43) Date of publication of application: **21.10.2026**
(21) Application number: 24904177.3
(22) Date of filing: 05.12.2024
(51) Int. Cl.: C07K 16/28, A61P 35/00, A61K 39/00

(54) **CANINE PDL-1 ANTIBODY AND USE THEREOF**

(30) Priority: 13.12.2023 KR 20230180613
(71) Applicant: Nodcure Inc., Gwangju 61186 (KR)
(72) Inventor: KIM, Dae Hee, Seoul 06190 (KR); KO, Hyun-Jeong, Seoul 06280 (KR); SONG, Min-Young, Chuncheon-si Gangwon-do 24264 (KR); CHO, Jaewon, Chuncheon-si Gangwon-do 24449 (KR); SONG, Yu-Jeong, Chuncheon-si Gangwon-do 24269 (KR); TAE, Nara, Wonju-si Gangwon-do 26386 (KR)
(74) Representative: Mewburn Ellis LLP
(86) International application number: PCT/KR2024/019867
(87) International publication number: WO 2025/127612

(57) **Abstract**

The present invention relates to a monoclonal antibody specific to canine PDL-1 or an antigen-binding fragment thereof, a pharmaceutical composition comprising same, and a use thereof. When using a novel canine PDL-1 antibody according to the present invention having high specificity for canine PDL-1 and having a tumor inhibitory effect in a canine osteosarcoma mouse model, a high therapeutic effect can be expected for canine cancers for which conventional immunotherapy has not been able to be performed.

## Description

### [Technical Field]

The present invention relates to a monoclonal antibody specific for PDL-1 or an antigen-binding fragment thereof, and more particularly, to a monoclonal antibody specific for canine PDL-1 or an antigen-binding fragment thereof, a pharmaceutical composition containing the same, and a use thereof.

### [Background Art]

Cancer is a disease that is widely prevalent in dogs as well as in humans, and occurs in various forms depending on factors such as breed and gender. Common tumors in dogs include histiocytoma, lipoma, adenoma, and mammary tumors. Current methods used to treat cancer in dogs include surgical removal of tumor tissue, radiation therapy, and chemotherapy.

The regulation of the immune system in the body involves complicated immune checkpoint mechanisms that control T lymphocyte function, antigen recognition, and the balance between stimulatory and inhibitory signals. Detection of tumor-specific neoantigens induced by mutations in tumor cells helps eliminate tumors and viral sources. However, some tumor cells evade attacks from the immune system by modulating the tumor microenvironment to induce immune tolerance or by escaping immune cell responses through immune editing. Alterations in immune checkpoint ligand/receptor binding have a significant impact on the activity of tumor-specific T lymphocytes. It has been reported that monoclonal antibodies that inhibit the function of PD-1 or PD-L1 ligands can enhance the activity of tumor-specific T lymphocytes and thereby provide anti-cancer effects, leading to significant advances in cancer therapy (Curiel TJ et al., Nature Medicine, 9:562, 2003).

PD-L1 (programmed death-ligand 1) has unique structural features including two Ig-like domains in the extracellular region, a transmembrane domain, and a short cytoplasmic domain lacking a known signaling motif. PD-L1, encoded by the CD274 gene, has approximately 20% amino acid sequence identity with B7.1 and B7.2, which belong to the B7 protein family. PD-L1 binds to PD-1, which is an immune checkpoint regulatory receptor expressed on various immune cells, thereby influencing T-cell immune activity. PD-L1 mediates immune protection against cytotoxic T lymphocyte (CTL) -mediated killing and regulates chronic immune responses through upregulation that interferes with immune protective pathways. PD-L1 may also interact with B7.1 and thereby exert potential effects on immune regulation, particularly in tumor immune evasion.

Although the therapeutic efficacy of immune checkpoint inhibitors in cancer treatment has been demonstrated in human oncology, cancer therapy using such approaches has not been extensively studied in the field of veterinary medicine. As a result, the lack of canine-specific therapeutic antibodies for immunotherapeutic strategies to treat cancer in dogs is a notable limitation.

Several studies have showed that PD-L1 expression is increased in dogs with tumors (Maekawa N et al., NPJ Precision Oncology, 5:10, 2021). In addition, blocking PD-1/PD-L1 interactions has been shown to enhance T-cell function in dogs with cancer (Choi JW et al., PLoS One, 15:e0235518, 2020). To date, none of the antibodies developed so far have been approved as a therapeutic agent for the treatment of cancer in dogs.

Accordingly, for canine cancer treatment using an immune checkpoint inhibitor, the present inventors selected scFvs (single-chain variable fragments) specific for canine PD-L1 from a synthetic scFv library using phage display technology, fused the selected scFvs with a human IgG1 constant region sequence to develop IgG-format antibodies, and identified the binding affinity and anti-cancer activity of the developed canine PD-L1-binding antibodies in both *in vitro* and *in vivo* models, thereby completing the present invention.

### [Disclosure]

It is an object of the present invention to provide an antibody against canine PDL-1, a pharmaceutical composition containing the same, and a method of treating tumors using the same.

In accordance with one aspect of the present invention, the above and other objects can be accomplished by the provision of a monoclonal antibody or an antigen-binding fragment thereof,
wherein the monoclonal antibody includes: a heavy chain variable region (V_{H}) including HCDR1 set forth in SEQ ID NO: 14, HCDR2 set forth in SEQ ID NO: 16, and HCDR3 set forth in SEQ ID NO: 18; and
a light chain variable region (V_{L}) including LCDR1 set forth in SEQ ID NO: 21, LCDR2 set forth in SEQ ID NO: 23, and LCDR3 set forth in SEQ ID NO: 25.

In accordance with another aspect, provided is a monoclonal antibody or an antigen-binding fragment thereof,
wherein the monoclonal antibody includes a heavy chain variable region (V_{H}) including HCDR1 set forth in SEQ ID NO: 28, SEQ ID NO: 42, SEQ ID NO: 56, SEQ ID NO: 70, or SEQ ID NO: 84; HCDR2 set forth in SEQ ID NO: 30, SEQ ID NO: 44, SEQ ID NO: 58, SEQ ID NO: 72, or SEQ ID NO: 86; and HCDR3 set forth in SEQ ID NO: 32, SEQ ID NO: 46, SEQ ID NO: 60, SEQ ID NO: 74, or SEQ ID NO: 88; and
a light chain variable region (V_{L}) including LCDR1 set forth in SEQ ID NO: 35, SEQ ID NO: 49, SEQ ID NO: 63, SEQ ID NO: 77, or SEQ ID NO: 91; LCDR2 set forth in SEQ ID NO: 37, SEQ ID NO: 51, SEQ ID NO: 65, SEQ ID NO: 79, or SEQ ID NO: 93; and LCDR3 set forth in SEQ ID NO: 39, SEQ ID NO: 53, SEQ ID NO: 67, SEQ ID NO: 81, or SEQ ID NO: 95.

In accordance with another aspect, provided is an isolated nucleic acid molecule including a nucleotide sequence encoding the heavy chain variable region (V_{H}) of the monoclonal antibody or the antigen-binding fragment thereof.

In accordance with another aspect, provided is an isolated nucleic acid molecule including a nucleotide sequence encoding the heavy chain variable region (V_{H}) of the monoclonal antibody or the antigen-binding fragment thereof.

In accordance with another aspect, provided is an isolated nucleic acid molecule including a nucleotide sequence encoding the light chain variable region (V_{L}) of the monoclonal antibody or the antigen-binding fragment thereof.

In accordance with another aspect, provided is a vector containing the isolated nucleic acid molecule.

In accordance with another aspect, provided is a host cell containing the isolated nucleic acid molecule or the vector.

In accordance with another aspect, provided is a pharmaceutical composition for treating a tumor containing: the monoclonal antibody or the antigen-binding fragment thereof; and a pharmaceutically acceptable carrier or excipient.

In accordance with another aspect, provided is a method of treating or preventing a tumor in a canine, including administering, to the canine having the tumor, the monoclonal antibody or the antigen-binding fragment thereof, or the pharmaceutical composition for treating a tumor.

In accordance with another aspect, provided is a method of treating or preventing a tumor including administering the monoclonal antibody or the antigen-binding fragment thereof, or the pharmaceutical composition for treating a tumor.

In accordance with another aspect, provided is a use of the monoclonal antibody or the antigen-binding fragment thereof, or the pharmaceutical composition for treating a tumor, for the treatment or prevention of a tumor.

In accordance with another aspect, provided is a use of the monoclonal antibody or the antigen-binding fragment thereof, or the pharmaceutical composition for treating a tumor, for the preparation of a drug for treating or preventing a tumor.

### [Description of Drawings]

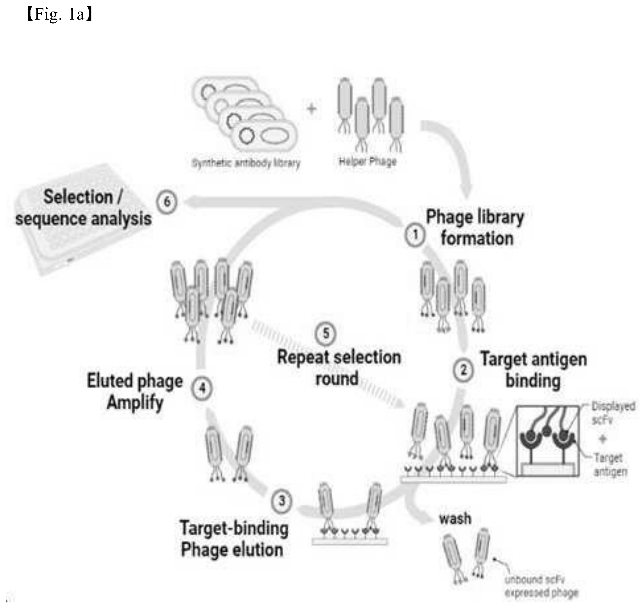

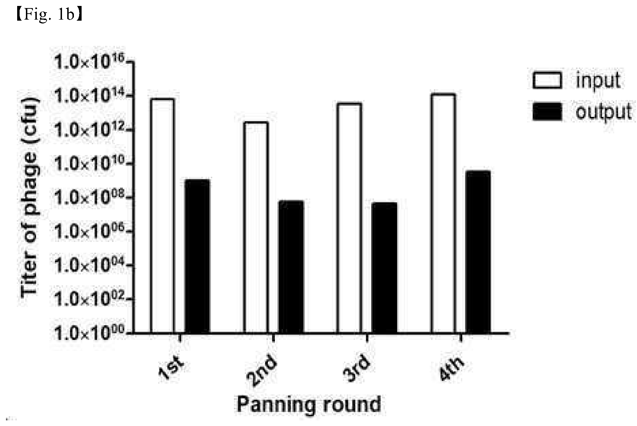

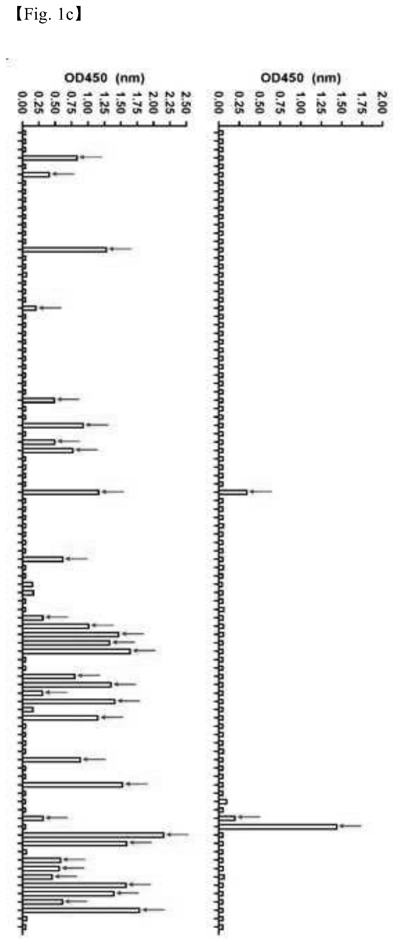

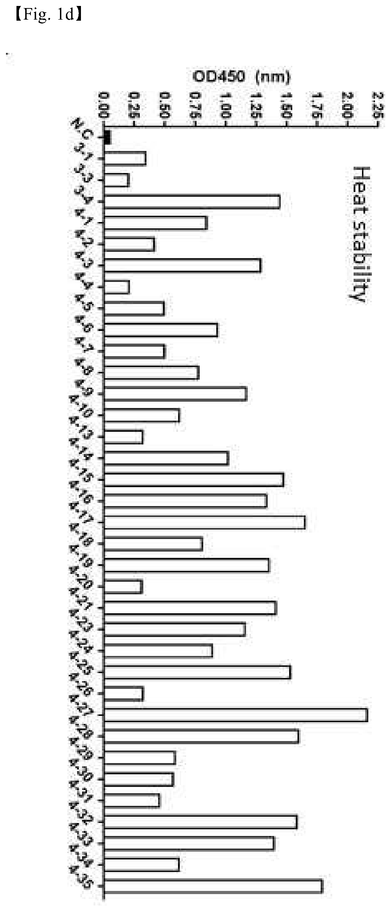

FIG. 1 shows a method of screening antibodies specific for canine PD-L1 and the results of identifying scFv clones against canine PD-L1, more specifically, FIG. 1A schematically illustrates a phage display method used in the present invention, FIG. 1B shows the titers obtained from four rounds of panning, wherein the titers were determined by the number of ER2738 colonies infected with phage, FIG. 1C shows the results of screening clones from the third and fourth panning output pools by scFv binding analysis in order to select specific binders to canine PD-L1, wherein the binding activity of 2 × periplasmic scFv clones to canine PD-L1 was determined by ELISA using TES periplasmic extraction, and FIG. 1D shows the results of analysis of the thermal stability activity of 35 scFv clones selected by the binding analysis, wherein OD at 450 nm was measured using scFvs heated at 70°C.

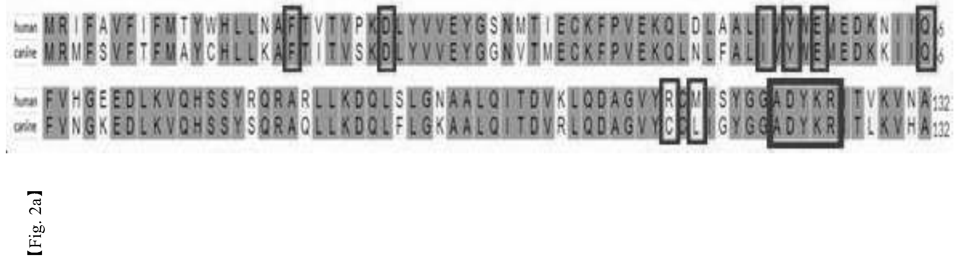

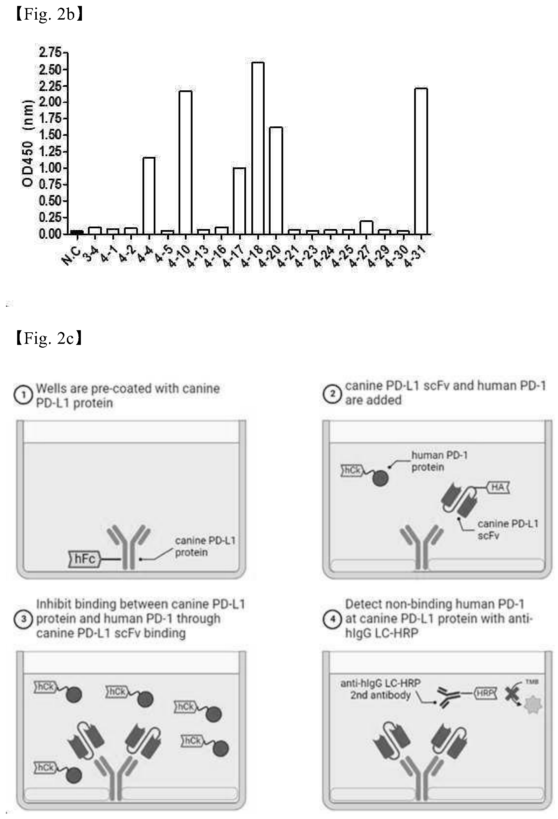

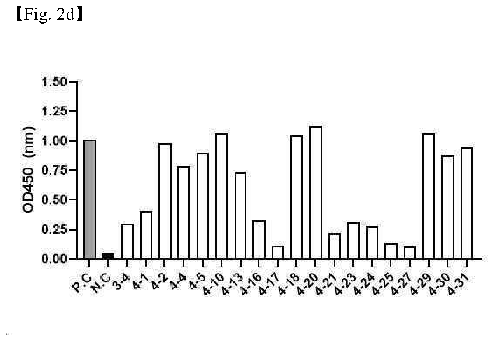

FIG. 2 shows the results of *in vitro* PD-1/PD-L1 interaction inhibition analysis and verification of the functional activity of scFvs, more specifically, FIG. 2A shows a comparison of the amino acid sequences of human PD-L1 and canine PD-L1, wherein purple highlights indicate regions where the amino acid sequences of the two species are identical, and the red box indicates the amino acid sequence of canine PD-L1 in a region known to be important for the binding of human PD-1 to human PD-L1, and FIG. 2B shows the results of detecting the binding of 19 scFvs to human PD-L1 by ELISA, wherein N.C. represents a sample treated with only the secondary antibody, FIG. 2C is a schematic diagram illustrating a method of determining whether or not the 19 scFvs inhibit the binding of human PD-1 to canine PD-L1 using competitive ELISA, FIG. 2D is a graph showing ELISA results indicating the inhibitory activity of cPD-L1 scFvs, wherein P.C. represents a sample indicating the binding ability between canine PD-L1 protein and human PD-1 protein, and N.C. represents a sample in which the canine PD-L1 protein is coated with only the secondary antibody.

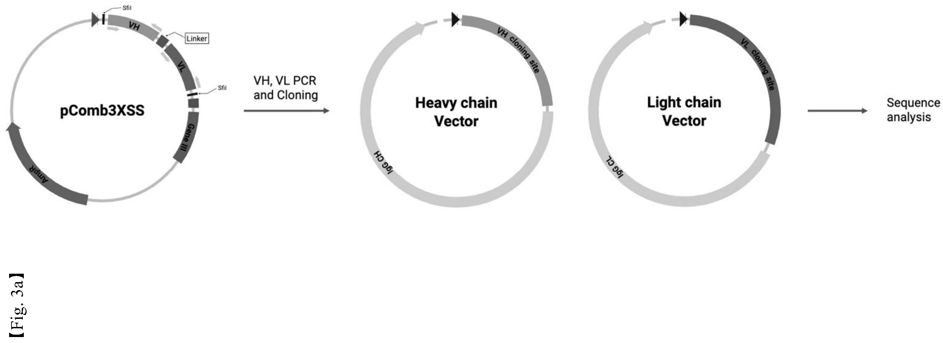

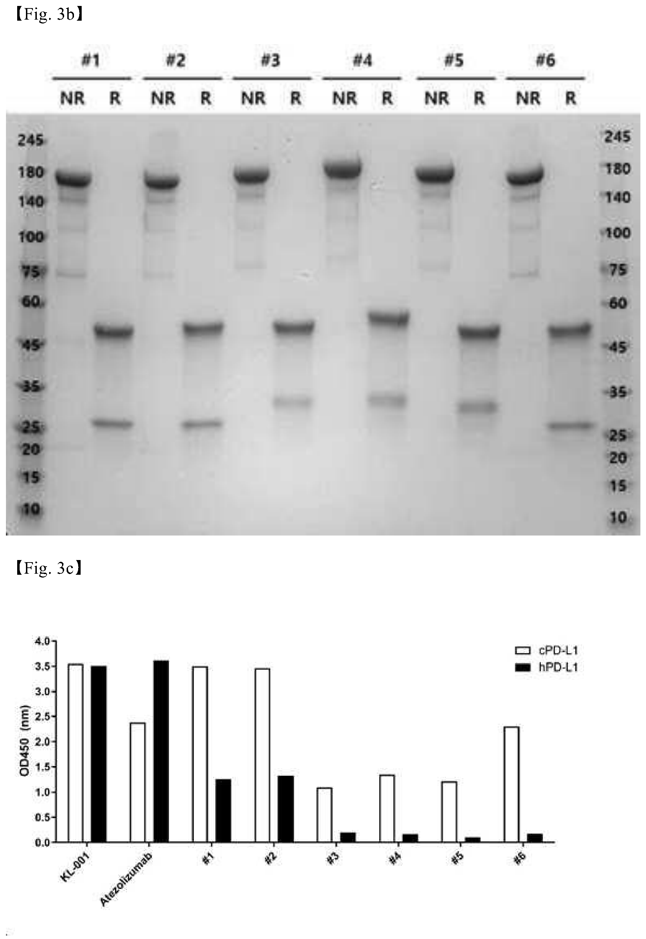

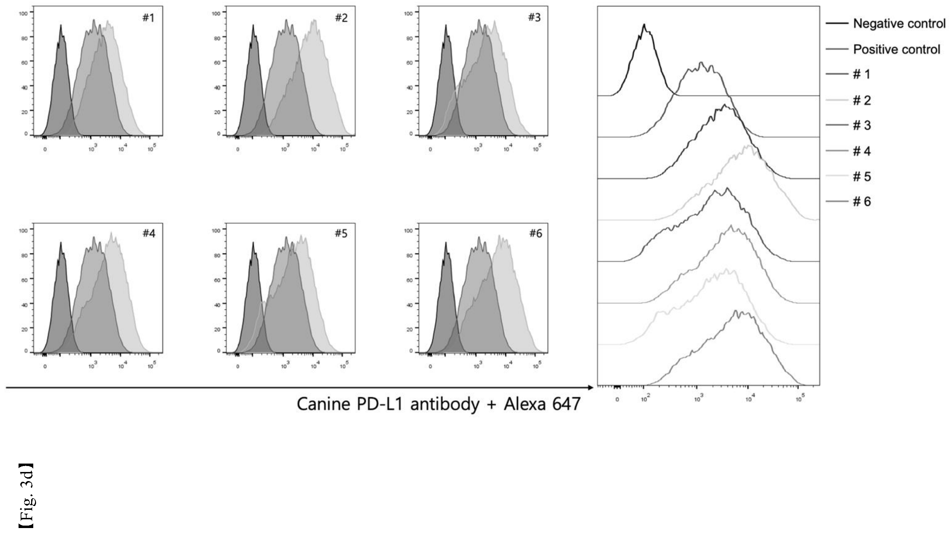

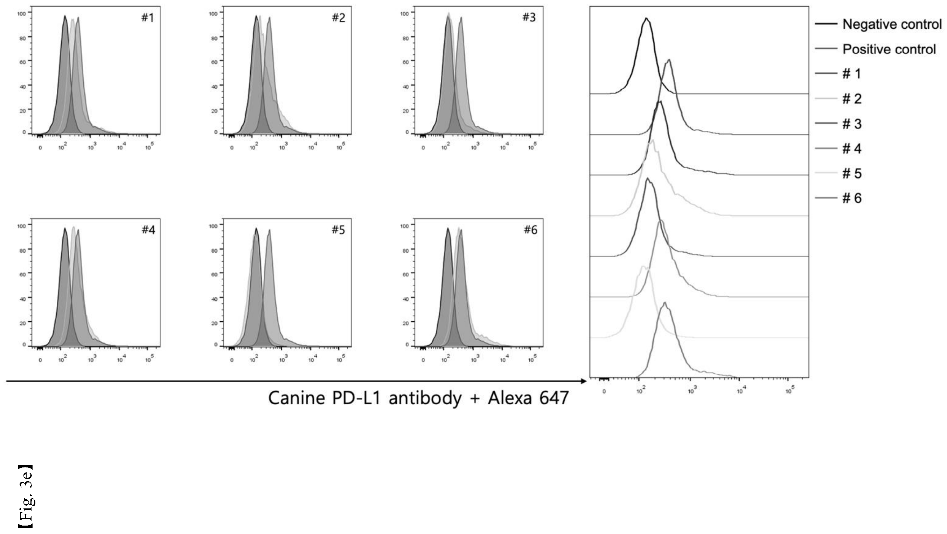

FIG. 3 shows the results of conversion of the selected scFvs into IgG format and verification of the function of the converted IgGs, more specifically, FIG. 3A is an overall schematic diagram illustrating a method of converting scFv clones having independent sequences into IgG, wherein the heavy chain DNA and light chain DNA fragments of the antibody were ligated into vectors capable of expression in FreeStyle^{™} 293-F cells and the FreeStyle^{™} 293-F cells were infected with plasmid samples using a two-vector system, FIG. 3B shows the results of purification of IgG from FreeStyle^{™} 293-F cells infected using the two-vector system and identification of the purified IgG by electrophoresis on an SDS-PAGE gel, FIG. 3C shows the results of determination of the binding affinity of anti-canine PD-L1 antibodies to canine PD-L1 and human PD-L1 by ELISA, wherein KL-001 is a positive antibody that binds to both canine and human PD-L1, whereas atezolizumab is a positive antibody that binds to human PD-L1, and FIGS. 3D and 3E show the results of measurement of the binding activity of six antibodies by flow cytometry after treatment of canine cell lines expressing canine PD-L1 with KL-001 (red line), anti-canine PD-L1 antibodies (blue line), or a control antibody (secondary anti-human Fc antibody, black line), more specifically, FIG. 3D shows the results for DH82 cells, and FIG. 3E shows the results for D17 cells wherein flow cytometry was detected using Alexa 488-conjugated anti-human Fc or Alexa 647-conjugated anti-human Fc as secondary fluorescent antibodies.

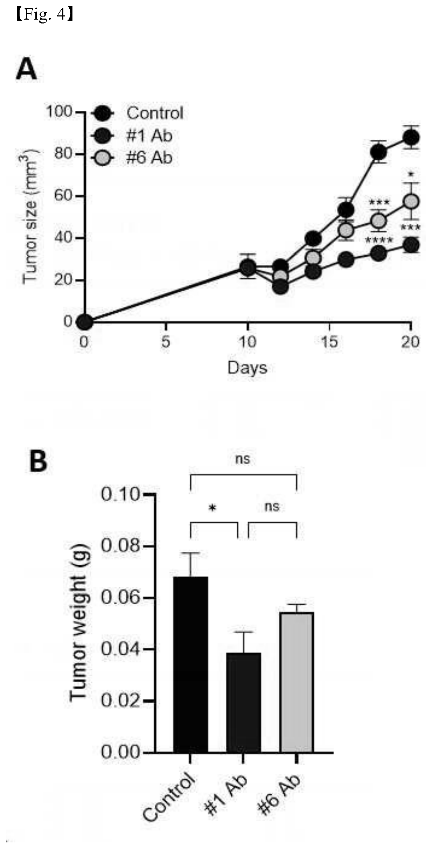

FIG. 4 shows the results of evaluation of the *in vivo* anti-tumor effect of the canine PD-L1 antibody in a canine osteosarcoma mouse model, more specifically, FIG. 4A shows that canine tumor cells (D17 cells, 4×10⁶ cells/mouse) were implanted into the left flank of mice and observed for 20 days, ten days after tumor inoculation, canine PBMCs were intravenously injected (8×10⁶ cells/mouse), antibody #1 or antibody #6 was administered intraperitoneally at a concentration of 1 mg/ml, and tumor growth was measured every two days (*p<0.05, ***p<0.001, ****p<0.0001; one-way ANOVA; n=5/group), and FIG. 4B shows the results of measurement of tumor mass after euthanizing all mouse groups and collecting tumors, wherein *n.s.; not significant and *p<0.05 (one-way ANOVA; n=5/group) and the data were obtained from three independent experiments.

### [Best Model

Unless defined otherwise, all technical and scientific terms used herein have the same meanings as appreciated by those skilled in the field to which the present invention pertains. In general, the nomenclature used herein is well-known in the art and is ordinarily used.

Immunotherapy has emerged as a breakthrough approach in human cancer treatment, particularly targeting immune checkpoint molecules such as PD-1 and PD-L1. Although the efficacy thereof was proven in human oncology, this innovative approach has been insufficiently studied in the field of veterinary medicine, and thus canine-specific therapeutic antibodies to utilize immunotherapeutic strategies in the treatment of cancer in dogs have not been available.

In the present invention, a therapeutic antibody against canine PDL-1 was developed. In the present invention, phage display technology was used to isolate single-chain variable fragments (scFvs) that specifically bind to canine PD-L1 using a synthetic antibody library. Through phage display screening of the scFv library, more than 35 high-affinity clones showing high ELISA signals, thermal stability, and unique sequences in the complementarity-determining regions (CDRs) of the heavy and light chains were selected. 19 scFv antibodies having independent CDR sequences were identified therefrom. In order to evaluate the function of the selected 19 scFv antibodies, whether or not canine PD-L1 is associated with human PD-1/PD-L1 interaction was evaluated. First, the amino acid sequence similarity between human PD-L1 and canine PD-L1 was 76.04%, as confirmed in UniProt. Binding affinity and binding capability to human PD-L1 were determined by direct ELISA. Functional analysis for PD-1/PD-L1 inhibition showed that the antibodies effectively blocked PD-1/PD-L1 interaction. Therefore, these candidate antibodies demonstrated the potential for development of novel therapeutic antibodies targeting canine PD-1/PD-L1 interaction.

In the present invention, the scFv format of the top six candidates having high binding affinity to canine PD-L1 antigen was converted into an IgG format.

In addition, it was evaluated whether or not the candidate antibodies converted into the IgG format still retained the functional activity thereof against the target antigen. As a result, all candidate antibodies in the IgG format exhibited sustained binding affinity to canine PD-L1. Cell-based *in vitro* analyses were performed using canine-derived cells known to express PD-L1. The result showed that some candidate antibodies still retained binding affinity to canine PD-L1 on the cell surface. Furthermore, tumor xenograft experiments in mice demonstrated a significant reduction in tumor growth, which indicates that the selected canine PD-L1 antibodies exert similar functional activity *in vivo.*

The amino acid sequences and nucleotide sequences of the heavy chain variable regions (V_{H}) and light chain variable regions (V_{L}) of the six canine PDL-1 antibodies according to the present invention are shown in Tables 1 and 14.

In addition, the Kabat sequences of the heavy chain variable regions (V_{H}) and light chain variable regions (V_{L}) of the selected six antibodies are shown in Tables 2 to 13.

**[Table 1]**

| Amino acid sequences of the VH and VL of six canine PD-L1 antibodies according to the present invention | | |
|---|---|---|
| Antibody | VH/VL (SEQ ID NO:) | Amino acid sequence |
| #1 | Heavy chain variable region (VH) (SEQ ID NO: 1) | EVQLLESGGGLVQPGGSLRLSCAASGFTFSGYDMSWVRQ APGKGLEWVSAISHDGGSIYYADSVKGRFTISRDNSKNTL YLQMNSLRAEDTAVYYCAKGAWPRPHWIFDYWGQGTL VTVSS |
| | Light chain variable region (VL) (SEQ ID NO: 2) | QSVLTQPPSASGTPGQRVTISCSGSSSNIGSNAVSWYQQLP GTAPKLLIYANSHRPSGVPDRFSGSKSGTSASLAISGLRSE DEADYYCAAWDDSLNGYVFGGGTKLTVL |
| #2 | Heavy chain variable region (VH) (SEQ ID NO:3) | EVQLLESGGGLVQPGGSLRLSCAASGFTFSDYDMSWVRQ APGKGLEWVSGTSYNGGSKYYADSVKGRFTISRDNSKNT LYLQMNSLRAEDTAVYYCAKDAWPIPRTHFDYWGQGTL VTVSS |
| | Light chain variable region (VL) (SEQ ID NO:4) | QSVLTQPPSASGTPGQRVTISCSGSSSNIGSNTFNWYQQLP GTAPKLLIYADSHRPSGVPDRFSGSKSGTSASLAISGLRSE DEADYYCGTWDSSLSAYVFGGGTKLTVL |
| #3 | Heavy chain variable region (VH) (SEQ ID NO: 5) | EVQLLESGGGLVQPGGSLRLSCAASGFTFSDYYMSWVRQ APGKGLEWVSGISPDDSSTYYADSVKGRFTISRDNSKNTL YLQMNSLRAEDTAVYYCAKDGWLPPRSLFDYWGQGTLV TVSS |
| | Light chain variable region (VL) (SEQ ID NO: 6) | QSVLTQPPSASGTPGQRVTISCTGSSSNIGSNNVTWYQQLP GTAPKLLIYSDNQRPSGVPDRFSGSKSGTSASLAISGLRSE DEADYYCGAWDDSLSGYVFGGGTKLTVL |
| #4 | Heavy chain variable region (VH) (SEQ ID NO: 7) | EVQLLESGGGLVQPGGSLRLSCAASGFTFSGYDMSWVRQ APGKGLEWVSGISHNSSNTYYADSVKGRFTISKDNSKNTL YLQMNSLRAEDTAVYYCAKGLLSHHMAYFDYWGQGTL VTVSS |
| | Light chain variable region (VL) (SEQ ID NO: 8) | QSVLTQPPSASGTPGQRVTISCTGSSSNIGSNNVTWYQQLP GTAPKLLIYSDSHRPSGVPDRFSGSKSGTSASLAISGLQSE DEADYYCGSWDYSLSAYVFGGGTKLTVL |
| #5 | Heavy chain variable region (VH) (SEQ ID NO: 9) | EVQLLESGGGLVQPGGSLRLSCAASGFTFSDYYMSWVRQ APGKGLEWVSGISPDDSSTYYADSVKGRFTISRDNSKNTL YLQMNSLRAEDTAVYYCAKDGWLPPRSLFDYWGQGTLV TVSS |
| | Light chain variable region (VL) (SEQ ID NO: 10) | QSVLTQPPSASGTPGQRVTISCTGSSSNIGSNNVTWYQQLP GTAPKLLIYSDNQRPSGVPDRFSGSKSGTSASLAISGLRSE DEADYYCGAWDDSLSGYVFGGGTKLTVL |
| #6 | Heavy chain variable region (VH) (SEQ ID NO: 11) | EVQLLESGGGLVQPGGSLRLSCAASGFTFSSYDMSWVRQ APGKGLEWVSAISYDNGNTYYADSVKGRFTISRDNSKNT LYLQMNSLRAEDTAVYYCARDVWPISNGIFDYWGQGTL VTVSS |
| | Light chain variable region (VL) (SEQ ID NO: (12) | QSVLTQPPSASGTPGQRVTISCSGSSSNIGSNDVTWYQQLP GTAPKLLIYANSHRPSGVPDRFSGSKSGTSASLAISGLRSE DEADYYCAAWDASLNGYVFGGGTKLTVL |

**[Table 2]**

| Kabat sequence of the heavy chain variable region (VH) of antibody #1 | | | | |
|---|---|---|---|---|
| Region | Sequence Fragment | Residues | SEQ ID NO: | Length |
| HFR1 | EVQLLESGGGLVQPGGSLRLSCAASGFT FS | 1 - 30 | 13 | 30 |
| CDR-H1 | GYDMS | 31 - 35 | 14 | 5 |
| HFR2 | WVRQAPGKGLEWVS | 36 - 49 | 15 | 14 |
| CDR-H2 | AISHDGGSIYYADSVKG | 50 - 66 | 16 | 17 |
| HFR3 | RFTISRDNSKNTLYLQMNSLRAEDTAVY YCAK | 67 - 98 | 17 | 32 |
| CDR-H3 | GAWPRPHWIFDY | 99 - 110 | 18 | 12 |
| HFR4 | WGQGTLVTVSS | 111 - 121 | 19 | 11 |
| | | | | 121 |

**[Table 3]**

| Kabat sequence of the light chain variable region (VL) of antibody #1 | | | | |
|---|---|---|---|---|
| Region | Sequence Fragment | Residues | SEQ ID NO: | Length |
| LFR1 | QSVLTQPPSASGTPGQRVTISC | 1 - 22 | 20 | 22 |
| CDR-L1 | SGSSSNIGSNAVS | 23 - 35 | 21 | 13 |
| LFR2 | WYQQLPGTAPKLLIY | 36 - 50 | 22 | 15 |
| CDR-L2 | ANSHRPS | 51 - 57 | 23 | 7 |
| LFR3 | GVPDRFSGSKSGTSASLAISGLRSEDEADYYC | 58 - 89 | 24 | 32 |
| CDR-L3 | AAWDDSLNGYV | 90 - 100 | 25 | 11 |
| LFR4 | FGGGTKLTVL | 101 - 110 | 26 | 10 |
| | | | | 110 |

**[Table 4]**

| Kabat sequence of the heavy chain variable region (VH) of antibody #2 | | | | |
|---|---|---|---|---|
| Region | Sequence Fragment | Residues | SEQ ID NO: | Length |
| HFR1 | EVQLLESGGGLVQPGGSLRLS CAASGFTFS | 1 - 30 | 27 | 30 |
| CDR-H1 | DYDMS | 31 - 35 | 28 | 5 |
| HFR2 | WVRQAPGKGLEWVS | 36 - 49 | 29 | 14 |
| CDR-H2 | GTSYNGGSKYYADSVKG | 50 - 66 | 30 | 17 |
| HFR3 | RFTISRDNSKNTLYLQMNSLR AEDTAVYYCAK | 67 - 98 | 31 | 32 |
| CDR-H3 | DAWPIPRTHFDY | 99 - 110 | 32 | 12 |
| HFR4 | WGQGTLVTVSS | 111 - 121 | 33 | 11 |
| | | | | 121 |

**[Table 5]**

| Kabat sequence of the light chain variable region (VL) of antibody #2 | | | | |
|---|---|---|---|---|
| Region | Sequence Fragment | Residues | SEQ ID NO: | Length |
| LFR1 | QSVLTQPPSASGTPGQRVTIS C | 1 - 22 | 34 | 22 |
| CDR-L1 | SGSSSNIGSNTFN | 23 - 35 | 35 | 13 |
| LFR2 | WYQQLPGTAPKLLIY | 36 - 50 | 36 | 15 |
| CDR-L2 | ADSHRPS | 51 - 57 | 37 | 7 |
| LFR3 | GVPDRFSGSKSGTSASLAISG LRSEDEADYYC | 58 - 89 | 38 | 32 |
| CDR-L3 | GTWDSSLSAYV | 90 - 100 | 39 | 11 |
| LFR4 | FGGGTKLTVL | 101 - 110 | 40 | 10 |
| | | | | 110 |

**[Table 6]**

| Kabat sequence of the heavy chain variable region (VH) of antibody #3 | | | | |
|---|---|---|---|---|
| Region | Sequence Fragment | Residues | SEQ ID NO: | Length |
| HFR1 | EVQLLESGGGLVQPGGSLRLSCAASG FTFS | 1 - 30 | 41 | 30 |
| CDR-H1 | DYYMS | 31 - 35 | 42 | 5 |
| HFR2 | WVRQAPGKGLEWVS | 36 - 49 | 43 | 14 |
| CDR-H2 | GISPDDSSTYYADSVKG | 50 - 66 | 44 | 17 |
| HFR3 | RFTISRDNSKNTLYLQMNSLRAEDTA VYYCAK | 67 - 98 | 45 | 32 |
| CDR-H3 | DGWLPPRSLFDY | 99 - 110 | 46 | 12 |
| HFR4 | WGQGTLVTVSS | 111 - 121 | 47 | 11 |
| | | | | 121 |

**[Table 7]**

| Kabat sequence of the light chain variable region (VL) of antibody #3 | | | | |
|---|---|---|---|---|
| Region | Sequence Fragment | Residues | SEQ ID NO: | Length |
| LFR1 | QSVLTQPPSASGTPGQRVTISC | 1 - 22 | 48 | 22 |
| CDR-L1 | TGSSSNIGSNNVT | 23 - 35 | 49 | 13 |
| LFR2 | WYQQLPGTAPKLLIY | 36 - 50 | 50 | 15 |
| CDR-L2 | SDNQRPS | 51 - 57 | 51 | 7 |
| LFR3 | GVPDRFSGSKSGTSASLAISGL RSEDEADYYC | 58 - 89 | 52 | 32 |
| CDR-L3 | GAWDDSLSGYV | 90 - 100 | 53 | 11 |
| LFR4 | FGGGTKLTVL | 101 - 110 | 54 | 10 |
| | | | | 110 |

**[Table 8]**

| Kabat sequence of the heavy chain variable region (VH) of antibody #4 | | | | |
|---|---|---|---|---|
| Regio n | Sequence Fragment | Residues | SEQ ID NO: | Length |
| HFR1 | EVQLLESGGGLVQPGGSLRLSCA ASGFTFS | 1 - 30 | 55 | 30 |
| CDR-H1 | GYDMS | 31 - 35 | 56 | 5 |
| HFR2 | WVRQAPGKGLEWVS | 36 - 49 | 57 | 14 |
| CDR-H2 | GISHNSSNTYYADSVKG | 50 - 66 | 58 | 17 |
| HFR3 | RFTISKDNSKNTLYLQMNSLRAE DTAVYYCAK | 67 - 98 | 59 | 32 |
| CDR-H3 | GLLSHHMAYFDY | 99 - 110 | 60 | 12 |
| HFR4 | WGQGTLVTVSS | 111 - 121 | 61 | 11 |
| | | | | 121 |

**[Table 9]**

| Kabat sequence of the light chain variable region (VL) of antibody #4 | | | | |
|---|---|---|---|---|
| Region | Sequence Fragment | Residues | SEQ ID NO: | Length |
| LFR1 | QSVLTQPPSASGTPGQRVTISC | 1 - 22 | 62 | 22 |
| CDR-L1 | TGSSSNIGSNNVT | 23 - 35 | 63 | 13 |
| LFR2 | WYQQLPGTAPKLLIY | 36 - 50 | 64 | 15 |
| CDR-L2 | SDSHRPS | 51 - 57 | 65 | 7 |
| LFR3 | GVPDRFSGSKSGTSASLAISGL QSEDEADYYC | 58 - 89 | 66 | 32 |
| CDR-L3 | GSWDYSLSAYV | 90 - 100 | 67 | 11 |
| LFR4 | FGGGTKLTVL | 101 - 110 | 68 | 10 |
| | | | | 110 |

**[Table 10]**

| Kabat sequence of the heavy chain variable region (VH) of antibody #5 | | | | |
|---|---|---|---|---|
| Region | Sequence Fragment | Residues | SEQ ID NO: | Length |
| HFR1 | EVQLLESGGGLVQPGGSLRLSC AASGFTFS | 1 - 30 | 69 | 30 |
| CDR-H1 | DYYMS | 31 - 35 | 70 | 5 |
| HFR2 | WVRQAPGKGLEWVS | 36 - 49 | 71 | 14 |
| CDR-H2 | GISPDDSSTYYADSVKG | 50 - 66 | 72 | 17 |
| HFR3 | RFTISRDNSKNTLYLQMNSLRA EDTAVYYCAK | 67 - 98 | 73 | 32 |
| CDR-H3 | DGWLPPRSLFDY | 99 - 110 | 74 | 12 |
| HFR4 | WGQGTLVTVSS | 111 - 121 | 75 | 11 |
| | | | | 121 |

**[Table 11]**

| Kabat sequence of the light chain variable region (VL) of antibody #5 | | | | |
|---|---|---|---|---|
| Region | Sequence Fragment | Residues | SEQ ID NO: | Length |
| LFR1 | QSVLTQPPSASGTPGQRVTISC | 1 - 22 | 76 | 22 |
| CDR-L1 | TGSSSNIGSNNVT | 23 - 35 | 77 | 13 |
| LFR2 | WYQQLPGTAPKLLIY | 36 - 50 | 78 | 15 |
| CDR-L2 | SDNQRPS | 51 - 57 | 79 | 7 |
| LFR3 | GVPDRFSGSKSGTSASLAISGLRS EDEADYYC | 58 - 89 | 80 | 32 |
| CDR-L3 | GAWDDSLSGYV | 90 - 100 | 81 | 11 |
| LFR4 | FGGGTKLTVL | 101 - 110 | 82 | 10 |
| | | | | 110 |

**[Table 12]**

| Kabat sequence of the heavy chain variable region (VH) of antibody #6 | | | | |
|---|---|---|---|---|
| Region | Sequence Fragment | Residues | SEQ ID NO: | Length |
| HFR1 | EVQLLESGGGLVQPGGSLRLSC AASGFTFS | 1 - 30 | 83 | 30 |
| CDR-H1 | SYDMS | 31 - 35 | 84 | 5 |
| HFR2 | WVRQAPGKGLEWVS | 36 - 49 | 85 | 14 |
| CDR-H2 | AISYDNGNTYYADSVKG | 50 - 66 | 86 | 17 |
| HFR3 | RFTISRDNSKNTLYLQMNSLRAE DTAVYYCAR | 67 - 98 | 87 | 32 |
| CDR-H3 | DVWPISNGIFDY | 99 - 110 | 88 | 12 |
| HFR4 | WGQGTLVTVSS | 111 - 121 | 89 | 11 |
| | | | | 121 |

**[Table 13]**

| Kabat sequence of the light chain variable region (V_{L}) of antibody #6 | | | | |
|---|---|---|---|---|
| Region | Sequence Fragment | Residues | SEQ ID NO: | Length |
| LFR1 | QSVLTQPPSASGTPGQRVTISC | 1 - 22 | 90 | 22 |
| CDR-L1 | SGSSSNIGSNDVT | 23 - 35 | 91 | 13 |
| LFR2 | WYQQLPGTAPKLLIY | 36 - 50 | 92 | 15 |
| CDR-L2 | ANSHRPS | 51 - 57 | 93 | 7 |
| LFR3 | GVPDRFSGSKSGTSASLAISGLR SEDEADYYC | 58 - 89 | 94 | 32 |
| CDR-L3 | AAWDASLNGYV | 90 - 100 | 95 | 11 |
| LFR4 | FGGGTKLTVL | 101 - 110 | 96 | 10 |
| | | | | 110 |

**[Table 14]**

| Nucleotide sequences of the VH and VL of six canine PD-L1 antibodies according to the present invention | | |
|---|---|---|
| Antibody | VH/VL | Nucleotide sequence |
| | (SEQ ID NO:) | |
| #1 | Heavy chain variable region (VH) (SEQ ID NO: 97) | GAGGTGCAGCTGTTGGAGTCTGGGGGAGGCTTGGTAC AGCCTGGGGGGTCCCTGAGACTCTCCTGTGCAGCCTCT GGATTCACCTTTAGCGGTTATGATATGAGCTGGGTCCG CCAGGCTCCAGGGAAGGGGCTGGAGTGGGTCTCAGCG ATCTCTCATGATGGTGGTAGTATATATTACGCTGATTC TGTAAAAGGTCGGTTCACCATCTCCAGAGACAATTCCA AGAACACGCTGTATCTGCAAATGAACAGCCTGAGAGC CGAGGACACGGCCGTGTATTACTGTGCGAAAGGTGCTT GGCCTCGTCCGCATTGGATTTTCGACTACTGGGGCCAG GGTACACTGGTCACCGTGAGCTCA |
| | Light chain variable region (VL) (SEQ ID NO: 98) | CAGTCTGTGCTGACTCAGCCACCCTCAGCGTCTGGGAC CCCCGGGCAGAGGGTCACCATCTCTTGTAGTGGCTCTT CGTCTAATATTGGCAGTAATGCTGTCTCCTGGTACCAG CAGCTCCCAGGAACGGCCCCCAAACTCCTCATCTATGC TAATAGTCATCGGCCAAGCGGGGTCCCTGACCGATTCT CTGGCTCCAAGTCTGGCACCTCAGCCTCCCTGGCCATC AGTGGGCTCCGGTCCGAGGATGAGGCTGATTATTACTG TGCTGCTTGGGATGATAGCCTGAATGGTTATGTCTTCG GCGGAGGCACCAAGCTGACGGTCCTA |
| #2 | Heavy chain variable region (VH) (SEQ ID NO: 99) | CGGGGGGGGGGCAATAGGCAGAGCTCGTTTAGTGAC CGTCAGATCTCTAGAAGCTGGGTACTTGGCTCGAGCCA CCATGGAGACAGACACACTCCTGCTATGGGTACTGCTG CTCTGGGTACCAGGATCCACTGGTGAGGTGCAGCTGTT GGAGTCTGGGGGAGGCTTGGTACAGCCTGGGGGGTCC CTGAGACTCTCCTGTGCAGCCTCTGGATTCACCTTTAG CGATTATGATATGAGCTGGGTCCGCCAGGCTCCAGGGA AGGGGCTGGAGTGGGTCTCAGGGACCTCTTATAATGGT GGTAGTAAATATTACGCTGATTCTGTAAAAGGTCGGTT CACCATCTCCAGAGACAATTCCAAGAACACGCTGTATC TGCAAATGAACAGCCTGAGAGCCGAGGACACGGCCGT |
| | | GTATTACTGTGCGAAAGATGCTTGGCCGATTCCTAGGA CGCATTTCGACTACTGGGGCCAGGGTACACTGGTCACC GTGAGCTCAGCTAGCACCAAGGGCCCATCGGTCTTCCC CCTGGCACCCTCCTCCAAGAGCACCTCTGGGGGCACAG CGGCCCTGGGCTGCCTGGTCAAGGACTACTTCCCCGAA CCGGTGACGGTGTCGTGGAACTCAGGCGCCCTGACCA GCGGCGTGCACACCTTCGCGGCTGTCCTACAGTCCTCA GGACTCTACTCCCTCAGCAGCGTGGTGACCGTGCCCTC CAGCAGCTTAGGCACCCAGACCTACATCTGCAACGTGA ATCACAAGCCCAGCAACGCCAAG |
| | Light chain variable region (VL) (SEQ ID NO: 100) | TTGGGGAAGTTTTATAAGCAGAGCTCGTTTAGTGACCG TCAGATCTCTAGAAGCTGGGTACTTGGCTAGCCACCAT GGAGACAGACACACTCCTGCTATGGGTACTGCTGCTCT GGGTACCAGGATCCACTGGTCAGTCTGTGCTGACTCAG CCACCCTCAGCGTCTGGGACCCCCGGGCAGAGGGTCA CCATCTCTTGTAGTGGCTCTTCATCTAATATTGGCAGTA ATACTTTCAACTGGTACCAGCAGCTCCCAGGAACGGCC CCCAAACTCCTCATCTATGCTGATAGTCATCGGCCAAG CGGGGTCCCTGACCGATTCTCTGGCTCCAAGTCTGGCA CCTCAGCCTCCCTGGCCATCAGTGGGCTCCGGTCCGAG GATGAGGCTGATTATTACTGTGGTACTTGGGATTCTAG CCTGAGTGCTTATGTCTTCGGCGGAGGCACCAAGCTGA CGGTCCTACGTACGGTGGCTGCACCATCTGTCTTCATC TTCCCGCCATCTGATGAGCAGTTGAAATCTGGAACTGC CTCTGTTGTGTGCCTGCTGAATAACTTCTATCCCAGAG AGGCCAAAGTACAGTGGAAGGTGGATAACGCCCTCCA ATCGGGTAACTCCCAGGAGAGTGTCACAGAGCAGGAC AGCAAGGACAGCACCTACAGCCTCAGCAGCACCCTGA CGCTGAGCAAAGCAGACTACGAGAAACACAAAGTCTA CGCCTGCGAAGTCACCCATCAGGGCCTGAGTTCGCCC GTCACAAAGAGCTTCAACAGGGGAGAGTGT |
| #3 | Heavy chain variable region (VH) (SEQ ID NO: 101) | GCGGTTTTGGGAGCCATTAAGCAGAGCTCGTTTAGTGA CCGTCAGATCTCTAGAAGCTGGGTACTTGGCTCGAGCC ACCATGGAGACAGACACACTCCTGCTATGGGTACTGCT GCTCTGGGTACCAGGATCCACTGGTGAGGTGCAGCTGT TGGAGTCTGGGGGAGGCTTGGTACAGCCTGGGGGGTC CCTGAGACTCTCCTGTGCAGCCTCTGGATTCACCTTTA GCGATTATTATATGAGCTGGGTCCGCCAGGCTCCAGGG AAGGGGCTGGAGTGGGTCTCAGGGATCTCTCCTGATGA TAGTAGTACATATTACGCTGATTCTGTAAAAGGTCGGT TCACCATCTCCAGAGACAATTCCAAGAACACGCTGTAT CTGCAAATGAACAGCCTGAGAGCCGAGGACACGGCCG TGTATTACTGTGCGAAAGATGGTTGGCTTCCTCCGCGT AGTCTGTTCGACTACTGGGGCCAGGGTACACTGGTCAC CGTGAGCTCAGCTAGCACCAAGGGCCCATCGGTCTTCC CCCTGGCACCCTCCTCCAAGAGCACCTCTGGGGGCACA GCGGCCCTGGGCTGCCTGGTCAAGGACTACTTCCCCGA ACCGGTGACGGTGTCGTGGAACTCAGGCGCCCTGACC AGCGGCGTGCACACCTTCCCGGCTGTCCTACAGTCCTC AGGACTCTACTCCCTCAGCAGCGTGGTGACCGTGCCCT CCAGCAGCTTAGGCACCCAGACCTACATCTGCAACGTG AATCACAAGCCCAGCAACACCAAGGTGGACAAGAAAG TTGAGCCCAAATCTTGTGACAAAACTCACACATGCCCA CCGTGCCCAGCACCTGAACTCCTGGGGGGACCGTCAGT CTTCCTCTTCCCCCCAAAACCCAAGGACACCCTCATGA TCTCCCGGACCCCTGAGGTCACATGCGTGGTGGTGGAC GTGAGCCACGAAGACCCTGAGGTCAAGTTCAACTGGT ACGTGGACGGCGTGGAGGTGCATAATGCCAAGACAAA GCCGCGGGAGGAGCAGTACAACAGCACGTACCGTGTG GTCAGCGTCCTCACCGTCCTGCACCAGGACTGGCTGAA TGGCAAGGAGTACAAGTGCAAGGTCTCCAACAAAGCC CTCCCAGCCCCCATCGAGAAAACCATCTCCAAAGCCAA |
| | | AGGGCAGCCCCGAGAAACCAAGGTGTACCCCTGCCCC CATCCCGGGATGAACTGACAAAGAACCAGGTCAGCCT GACCTGCCTGGTCAAAGGTTTATCCCCGCAAAATCCC CCGGGAAATGGGGAAAAAAAGGGGACCGGAAAAA AAAAAAAAAAACCCCCCCCGGGGGGGGAACCCAGG GCCCCTTTTCCCTAAAAAACCCCCCGGGAAAAAAAA GGGGGGGGGAGGGGGGAAAAAATT |
| | Light chain variable region (VL) (SEQ ID NO: 102) | CCGATTTGGGACTAAATAGCAGAGCTCGTTTAGTGACC GTCAGATCTCTAGAAGCTGGGTACTTGGCTAGCCACCA TGGAGACAGACACACTCCTGCTATGGGTACTGCTGCTC TGGGTACCAGGATCCACTGGTCAGTCTGTGCTGACTCA GCCACCCTCAGCGTCTGGGACCCCCGGGCAGAGGGTC ACCATCTCTTGTACTGGCTCTTCATCTAATATTGGCAGT AATAATGTCACCTGGTACCAGCAGCTCCCAGGAACGG CCCCCAAACTCCTCATCTATTCTGATAATCAGCGGCCA AGCGGGGTCCCTGACCGATTCTCTGGCTCCAAGTCTGG CACCTCAGCCTCCCTGGCCATCAGTGGGCTCCGGTCCG AGGATGAGGCTGATTATTACTGTGGTGCTTGGGATGAT AGCCTGAGTGGTTATGTCTTTGGCGGAGGCACCAAGCT GACGGTCCTACGTACGGTGGCTGCACCATCTGTCTTCA TCTTCCCGCCATCTGATGAGCAGTTGAAATCTGGAACT GCCTCTGTTGTGTGCCTGCTGAATAACTTCTATCCCAG AGAGGCCAAAGTACAGTGGAAGGTGGATAACGCCCTC CAATCGGGTAACTCCCAGGAGAGTGTCACAGAGCAGG ACAGCAAGGACAGCACCTACAGCCTCAGCAGCACCCT GACGCTGAGCAAAGCAGACTACGAGAAACACAAAGTC TACGCCTGCGAAGTCACCCATCAGGGCCTGAGTTCGCC CGTCACAAAGAGCTTCAACAGGGGAGAGTGTTAATTCT AGCGGCCGCAGATCCAGACATGATAAGATACATTGAT GAGTTTGGACAAACCACAACTAGAATGCAGTGAAAAA AATGCTTTATTTGTGAAATTTGTGATGCTATTGCTTTAT |
| | | TTGTAACCATTATAAGCTGCAATAAACAAGTTAACAAC AACAATTGCATTCATTTTATGTTTCAGGTTCAGGGGGA GGTGTGGGAGGTTTTTTAAAGCAAGTAAAACCTCTAC AAATGTGGTATGGCTGATTATGATCCGGCTGCCTCGCG CGTTTCGGTGATGACGGTGAAAACCTCTGAAACATGCA GCTCCCGGAAACGGTCACAGCTTGTCTGTAAGCGGATG CCGGGAACAGAACAACCCCGTCAGGGCGCGTCACCGG GTGGTTGGCGGGGGGTCCGGGGCCCAAACCCTGAAGT CCAAATCCTAGAAGAATCAATCCCCGCCCCCGGAAGA AAATAAAACCTGGAATAGAAAATTTTTTGCCCCCTTTT TTCCGGGGGAGAGGGCGGGTATAACCCCTTTCTTTGGG TGGGGAAAAATTTTCCCACGGGGCCCCTTTCCCCTTTG AACCGGGGGGGGGAA |
| #4 | Heavy chain variable region (VH) (SEQ ID NO: 103) | TCGGGGGGGGGAGTTTATAGCAGAGCTCGTTTAGTGAC CGTCAGATCTCTAGAAGCTGGGTACTTGGCTCGAGCCA CCATGGAGACAGACACACTCCTGCTATGGGTACTGCTG CTCTGGGTACCAGGATCCACTGGTGAGGTGCAGCTGTT GGAGTCTGGGGGAGGCTTGGTACAGCCTGGGGGGTCC CTGAGACTCTCCTGTGCAGCCTCTGGATTCACCTTTAG CGGTTATGATATGAGCTGGGTCCGCCAGGCTCCAGGGA AGGGGCTGGAGTGGGTCTCAGGGATCTCTCATAATAGT AGTAATACATATTACGCTGATTCTGTAAAAGGTCGGTT CACCATCTCCAAAGACAATTCCAAGAACACGCTGTATC TGCAAATGAACAGCCTGAGAGCCGAGGACACGGCCGT GTATTACTGTGCGAAAGGTCTTTTGAGTCATCATATGG CGTATTTCGACTACTGGGGCCAGGGTACACTGGTCACC GTGAGCTCAGCTAGCACCAAGGGCCCATCGGTCTTCCC CCTGGCACCCTCCTCCAAGAGCACCTCTGGGGGCACAG CGGCCCTGGGCTGCCTGGTCAAGGACTACTTCCCCGAA CCGGTGACGGTGTCGTGGAACTCAGGCGCCCTGACCA GCGGCGTGCACACCTTCCCGGCTGTCCTACAGTCCTCA |
| | | GGACTCTACTCCCTCAGCAGCGTGGTGACCGTGCCCTC CAGCAGCTTAGGCACCCAGACCTACATCTGCAACGTGA ATCACAAGCCCAGCAACACCAAGGTGGACAAGAAAGT TGAGCCCAAATCTTGTGACAAAACTCACACATGCCCAC CGTGCCCAGCACCTGAACTCCTGGGGGGACCGTCAGTC TTCCTCTTCCCCCCAAAACCCAAGGACACCCTCATGAT CTCCCGGACCCCTGAGGTCACATGCGTGGTGGTGGACG TGAGCCACGAAGACCCTGAGGTCAAGTTCAACTGGTA CGTGGACGGCGTGGAGGTGCATAATGCCAAGACAAAG CCGCGGGAGGAGCAGTACAACAGCACGTACCGTGTGG TCAGCGTCCTCACCGTCCTGCACCAGGACTGGCTGAAT GGCAAGGAGTACAAGTGCAAGGTCTCCAACAAAGCCC TCCCAGCCCCCATCGAGAAAACCATCTCCAAAGCCAA AGGGCAGCCCCGAGAACCAGGTGTACACCCTGCCCC ATCCCGGGATGAACTGACCAAGAACCAGGTCAGCCTG ACCTGCCTGGTCAAAGGTTTATCCCACCGAAATCGCC CGGGGAAGGGGAAGAAAAGGGGGAACCGGAAAAAA ACAAAAAAACCCCCCCCCCTGGGTGAACCCCAAGGGC CCCTTCTTCCCTAAAAAAAAGCTCCCCCGGGGAGAAAA GAGAGGGGGGAACAGGGGAAAACCTTT |
| | Light chain variable region (VL) (SEQ ID NO: 104) | CCGATTGTGGGAATCATATAGCAGAGCTCGTTTAGTGA CCGTCAGATCTCTAGAAGCTGGGTACTTGGCTAGCCAC CATGGAGACAGACACACTCCTGCTATGGGTACTGCTGC TCTGGGTACCAGGATCCACTGGTCAGTCTGTGCTGACT CAGCCACCCTCAGCGTCTGGGACCCCCGGGCAGAGGG TCACCATCTCTTGTACTGGCTCTTCATCTAATATTGGCA GTAATAATGTCACCTGGTACCAGCAGCTCCCAGGAACG GCCCCCAAACTCCTCATCTATTCTGATAGTCATCGGCC AAGCGGGGTCCCTGACCGATTCTCTGGCTCCAAGTCTG GCACCTCAGCCTCCCTGGCCATCAGTGGGCTCCAGTCC GAGGATGAGGCTGATTATTACTGTGGTTCTTGGGATTA |
| | | TAGCCTGAGTGCTTATGTCTTCGGCGGAGGCACCAAGC TGACGGTCCTACGTACGGTGGCTGCACCATCTGTCTTC ATCTTCCCGCCATCTGATGAGCAGTTGAAATCTGGAAC TGCCTCTGTTGTGTGCCTGCTGAATAACTTCTATCCCAG AGAGGCCAAAGTACAGTGGAAGGTGGATAACGCCCTC CAATCGGGTAACTCCCAGGAGAGTGTCACAGAGCAGG ACAGCAAGGACAGCACCTACAGCCTCAGCAGCACCCT GACGCTGAGCAAAGCAGACTACGAGAAACACAAAGTC TACGCCTGCGAAGTCACCCATCAGGGCCTGAGTTCGCC CGTCACAAAGAGCTTCAACAGGGGAGAGTGTTAATTCT AGCGGCCGCAGATCCAGACATGATAAGATACATTGAT GAGTTTGGACAAACCACAACTAGAATGCAGTGAAAAA AATGCTTTATTTGTGAAATTTGTGATGCTATTGCTTTAT TTGTAACCATTATAAGCTGCAATAAACAAGTTAACAAC AACAATTGCATTCATTTTATGTTTCAGGTTCAGGGGGA GGTGGTGGGGAGGTTTTTTAAAGCAAGTAAAACCTCTA CAAATGTGGTATGGCTGATTATGATCCGGCTGCCTCGC GCGTTTCGGTGATGACGGTGAAAACCTCTGACACATGC AGCTCCCGGAAAACGGTCACAGCTTGTCTGTAAACGG ATGCCGGGAACAGAACAACCCCGTCAGGGCCCGTCAC CCGGGTGGTTGGCGGGTGGTCCGGGGCGCACCCCTGA AGGCCAACTCTAAAGGAATCAATCCCCCCCCCCGGAA GAAAATAAAACCTGAAAAAAGAAATTCTTGGCCCCTTT TTTCCGGGGGGGGGGGGGGGAACCCCCTCCCTTGTG GGGGGGAAAAATTTTCCCACGGGGGCCCCTTTCCCCAT TTAAAACCGGGGGGGGAAA |
| #5 | Heavy chain variable region (VH) (SEQ ID NO: 105) | CCCGGAGTTGGGAGCAATAGCAGAGCTCGTTTAGTGA CCGTCAGATCTCTAGAAGCTGGGTACTTGGCTCGAGCC ACCATGGAGACAGACACACTCCTGCTATGGGTACTGCT GCTCTGGGTACCAGGATCCACTGGTGAGGTGCAGCTGT TGGAGTCTGGGGGAGGCTTGGTACAGCCTGGGGGGTC |
| | | CCTGAGACTCTCCTGTGCAGCCTCTGGATTCACCTTTA GCGATTATTATATGAGCTGGGTCCGCCAGGCTCCAGGG AAGGGGCTGGAGTGGGTCTCAGGGATCTCTCCTGATGA TAGTAGTACATATTACGCTGATTCTGTAAAAGGTCGGT TCACCATCTCCAGAGACAATTCCAAGAACACGCTGTAT CTGCAAATGAACAGCCTGAGAGCCGAGGACACGGCCG TGTATTACTGTGCGAAAGATGGTTGGCTTCCTCCGCGT AGTCTGTTCGACTACTGGGGCCAGGGTACACTGGTCAC CGTGAGCTCAGCTAGCACCAAGGGCCCATCGGTCTTCC CCCTGGCACCCTCCTCCAAGAGCACCTCTGGGGGCACA GCGGCCCTGGGCTGCCTGGTCAAGGACTACTTCCCCGA ACCGGTGACGGTGTCGTGGAACTCAGGCGCCCTGACC AGCGGCGTGCACACCTTCCCGGCTGTCCTACAGTCCTC AGGACTCTACTCCCTCAGCAGCGTGGTGACCGTGCCCT CCAGCAGCTTAGGCACCCAGACCTACATCTGCAACGTG AATCACAAGCCCAGCAACACCAAGGTGGACAAGAAAG TTGAGCCCAAATCTTGTGACAAAACTCACACATGCCCA CCGTGCCCAGCACCTGAACTCCTGGGGGGACCGTCAGT CTTCCTCTTCCCCCCAAAACCCAAGGACACCCTCATGA TCTCCCGGACCCCTGAGGTCACATGCGTGGTGGTGGAC GTGAGCCACGAAGACCCTGAGGTCAAGTTCAACTGGT ACGTGGACGGCGTGGAGGTGCATAATGCCAAGACAAA GCCGCGGGAGGAGCAGTACAACAGCACGTACCGTGTG GTCAGCGTCCTCACCGTCCTGCACCAGGACTGGCTGAA TGGCAAGGAGTACAAGTGCAAGGTCTCCAACAAAGCC CTCCCAGCCCCCATCGAGAAAACCTTTTCCAAAGCCAA AGGGCACCCCCAGAAACCCAGGGGTAACCCTGCCCCC ATCCCGGGATAAATGAACAAGAACAAGTTAACCCTGA ACTGCCTGGGTAAAGGGTTCTTTCCCCGGAAATCGCCC GGGGAAGGGGGAAGAGAAGGGGGGCCCGGGAAAAAA |
| | | AATAAAAAAAACCCCCTCCCCGGGGGAAACCCAAGG GTCCTCTTTTCTCCAAAACCCCGGGGGAA |
| | Light chain variable region (VL) (SEQ ID NO: 106) | CCCGAATGGGGTGGCATATAAGCAGAGCTCGTTTAGTG ACCGTCAGATCTCTAGAAGCTGGGTACTTGGCTAGCCA CCATGGAGACAGACACACTCCTGCTATGGGTACTGCTG CTCTGGGTACCAGGATCCACTGGTCAGTCTGTGCTGAC TCAGCCACCCTCAGCGTCTGGGACCCCCGGGCAGAGG GTCACCATCTCTTGTACTGGCTCTTCATCTAATATTGGC AGTAATAATGTCACCTGGTACCAGCAGCTCCCAGGAAC GGCCCCCAAACTCCTCATCTATTCTGATAATCAGCGGC CAAGCGGGGTCCCTGACCGATTCTCTGGCTCCAAGTCT GGCACCTCAGCCTCCCTGGCCATCAGTGGGCTCCGGTC CGAGGATGAGGCTGATTATTACTGTGGTGCTTGGGATG ATAGCCTGAGTGGTTATGTCTTTGGCGGAGGCACCAAG CTGACGGTCCTACGTACGGTGGCTGCACCATCTGTCTT CATCTTCCCGCCATCTGATGAGCAGTTGAAATCTGGAA CTGCCTCTGTTGTGTGCCTGCTGAATAACTTCTATCCCA GAGAGGCCAAAGTACAGTGGAAGGTGGATAACGCCCT CCAATCGGGTAACTCCCAGGAGAGTGTCACAGAGCAG GACAGCAAGGACAGCACCTACAGCCTCAGCAGCACCC TGACGCTGAGCAAAGCAGACTACGAGAAACACAAAGT CTACGCCTGCGAAGTCACCCATCAGGGCCTGAGTTCGC CCGTCACAAAGAGCTTCAACAGGGGAGAGTGTTAATT CTAGCGGCCGCAGATCCAGACATGATAAGATACATTG ATGAGTTTGGACAAACCACAACTAGAATGCAGTGAAA AAAATGCTTTATTTGTGAAATTTGTGATGCTATTGCTTT ATTTGTAACCATTATAAGCTGCAATAAACAAGTTAACA ACAACAATTGCATTCATTTTATGTTTCAGGTTCAGGGG GAGGTGTGGGAGGTTTTTTAAAGCAAGTAAAACCTCTA CAAATGTGGTATGGCTGAATATGATCCGGCTGCCTCGC GCGTTTCGGTGATGACGGTGAAAACCTCTGAACCATGC |
| | | AGCTCCCGGAGAACGGTCACAGCTTTGTCTGTAAACGG AAGGCCGGGAACAAAACAACCCCGTCAGGGGCCGCTC CACCGGGTGGTTGGCGGGGGGCGGGGGCCCACCCCT GAAGGGCCAAACCTATAAAAAAAAATTCCCCCCCCGG AAAAAAAAAAAAACTGAAATAAAAAATTTCTGCCCCCT TTTTTCCGGGGGAA |
| #6 | Heavy chain variable region (VH) (SEQ ID NO: 107) | CCCGGTGGGGAAGCATTAGCAGAGCTCGTTTAGTGACC GTCAGATCTCTAGAAGCTGGGTACTTGGCTCGAGCCAC CATGGAGACAGACACACTCCTGCTATGGGTACTGCTGC TCTGGGTACCAGGATCCACTGGTGAGGTGCAGCTGTTG GAGTCTGGGGGAGGCTTGGTACAGCCTGGGGGGTCCC TGAGACTCTCCTGTGCAGCCTCTGGATTCACCTTTAGC AGTTATGATATGAGCTGGGTCCGCCAGGCTCCAGGGA AGGGGCTGGAGTGGGTCTCAGCGATCTCTTATGATAAT GGTAATACATATTACGCTGATTCTGTAAAAGGTCGGTT CACCATCTCCAGAGACAATTCCAAGAACACGCTGTATC TGCAAATGAACAGCCTGAGAGCCGAGGACACGGCCGT GTATTACTGTGCGAGAGATGTTTGGCCGATTTCTAATG GGATTTTCGACTACTGGGGCCAGGGTACACTGGTCACC GTGAGCTCAGCTAGCACCAAGGGCCCATCGGTCTTCCC CCTGGCACCCTCCTCCAAGAGCACCTCTGGGGGCACAG CGGCCCTGGGCTGCCTGGTCAAGGACTACTTCCCCGAA CCGGTGACGGTGTCGTGGAACTCAGGCGCCCTGACCA GCGGCGTGCACACCTTCCCGGCTGTCCTACAGTCCTCA GGACTCTACTCCCTCAGCAGCGTGGTGACCGTGCCCTC CAGCAGCTTAGGCACCCAGACCTACATCTGCAACGTGA ATCACAAGCCCAG |
| | Light chain variable region (VL) | AGGTTGAAGTCTTATAAGCAGAGCTCGTTTAGTGAACC GTCAGATCTCTAGAAGCTGGGTACTTGGCTAGCCACCA TGGAGACAGACACACTCCTGCTATGGGTACTGCTGCTC TGGGTACCAGGATCCACTGGTCAGTCTGTGCTGACTCA |
| | (SEQ ID NO: 108) | GCCACCCTCAGCGTCTGGGACCCCCGGGCAGAGGGTC ACCATCTCTTGTAGTGGCTCTTCATCTAATATTGGCAGT AATGATGTCACCTGGTACCAGCAGCTCCCAGGAACGG CCCCCAAACTCCTCATCTATGCTAATAGTCATCGGCCA AGCGGGGTCCCTGACCGATTCTCTGGCTCCAAGTCTGG CACCTCAGCCTCCCTGGCCATCAGTGGGCTCCGGTCCG AGGATGAGGCTGATTATTACTGTGCTGCTTGGGATGCT AGCCTGAATGGTTATGTCTTCGGCGGAGGCACCAAGCT GACGGTCCTACGTACGGTGGCTGCACCATCTGTCTTCA TCTTCCCGCCATCTGATGAGCAGTTGAAATCTGGAACT GCCTCTGTTGTGTGCCTGCTGAATAACTTCTATCCCAG AGAGGCCAAAGTACAGTGGAAGGTGGATAACGCCCTC CAATCGGGTAACTCCCAGGAGAGTGTCACAGAGCAGG ACAGCAAGGACAGCACCTACAGCCTCAGCAGCACCCT GACGCTGAGCAAAGCAGACTACGAGAAACACAAAGTC TACGCCTGCGAAGTCACCCATCAGGGCCTGAGTTCGCC CGTCACAAAGAGCTTCAACAG |

Therefore, in one aspect, the present invention is directed to a monoclonal antibody or an antigen-binding fragment thereof,
wherein the monoclonal antibody includes: a heavy chain variable region (V_{H}) including HCDR1 set forth in SEQ ID NO: 14, HCDR2 set forth in SEQ ID NO: 16, and HCDR3 set forth in SEQ ID NO: 18; and
a light chain variable region (V_{L}) including LCDR1 set forth in SEQ ID NO: 21, LCDR2 set forth in SEQ ID NO: 23, and LCDR3 set forth in SEQ ID NO: 25.

In the present invention, the amino acid sequence of the heavy chain variable region (V_{H}) of the monoclonal antibody or the antigen-binding fragment thereof may be represented by SEQ ID NO: 1, and the amino acid sequence of the light chain variable region (V_{L}) of the monoclonal antibody or the antigen-binding fragment thereof may be represented by SEQ ID NO: 2.

In the present invention, the monoclonal antibody or the antigen-binding fragment thereof may be selected from Fab, Fab', F(ab')₂, Fd, Fv, dAb, a single-chain antibody, a humanized antibody, a chimeric antibody, and a diabody, wherein the single-chain antibody may be an scFv.

In the present invention, the monoclonal antibody or the antigen-binding fragment thereof may specifically bind to PDL-1, wherein the PDL-1 may be derived from a canine or a human.

In another aspect, the present invention is directed to a monoclonal antibody or an antigen-binding fragment thereof,
wherein the monoclonal antibody includes a heavy chain variable region (V_{H}) including HCDR1 set forth in SEQ ID NO: 28, SEQ ID NO: 42, SEQ ID NO: 56, SEQ ID NO: 70, or SEQ ID NO: 84; HCDR2 set forth in SEQ ID NO: 30, SEQ ID NO: 44, SEQ ID NO: 58, SEQ ID NO: 72, or SEQ ID NO: 86; and HCDR3 set forth in SEQ ID NO: 32, SEQ ID NO: 46, SEQ ID NO: 60, SEQ ID NO: 74, or SEQ ID NO: 88; and
a light chain variable region (V_{L}) including LCDR1 set forth in SEQ ID NO: 35, SEQ ID NO: 49, SEQ ID NO: 63, SEQ ID NO: 77, or SEQ ID NO: 91; LCDR2 set forth in SEQ ID NO: 37, SEQ ID NO: 51, SEQ ID NO: 65, SEQ ID NO: 79, or SEQ ID NO: 93; and LCDR3 set forth in SEQ ID NO: 39, SEQ ID NO: 53, SEQ ID NO: 67, SEQ ID NO: 81, or SEQ ID NO: 95.

In the present invention, the monoclonal antibody or the antigen-binding fragment thereof may be selected from the group consisting of:
(i) a monoclonal antibody or an antigen-binding fragment thereof including a heavy chain variable region (V_{H}) having an amino acid sequence of SEQ ID NO: 3 and a light chain variable region (V_{L}) having an amino acid sequence of SEQ ID NO: 4;
(ii) a monoclonal antibody or an antigen-binding fragment thereof including a heavy chain variable region (V_{H}) having an amino acid sequence of SEQ ID NO: 5 and a light chain variable region (V_{L}) having an amino acid sequence of SEQ ID NO: 6;
(iii) a monoclonal antibody or an antigen-binding fragment thereof including a heavy chain variable region (V_{H}) having an amino acid sequence of SEQ ID NO: 7 and a light chain variable region (V_{L}) having an amino acid sequence of SEQ ID NO: 8;
(iv) a monoclonal antibody or an antigen-binding fragment thereof including a heavy chain variable region (V_{H}) having an amino acid sequence of SEQ ID NO: 9 and a light chain variable region (V_{L}) having an amino acid sequence of SEQ ID NO: 10; and
(v) a monoclonal antibody or an antigen-binding fragment thereof including a heavy chain variable region (V_{H}) having an amino acid sequence of SEQ ID NO: 11 and a light chain variable region (V_{L}) having an amino acid sequence of SEQ ID NO: 12.

In another aspect, the present is directed to an isolated nucleic acid molecule including a nucleotide sequence encoding the heavy chain variable region (V_{H}) of the monoclonal antibody or the antigen-binding fragment thereof.

In the present invention, the monoclonal antibody or the antigen-binding fragment thereof may be selected from Fab, Fab', F(ab')₂, Fd, Fv, dAb, a single-chain antibody, a humanized antibody, a chimeric antibody, and a diabody, wherein the single-chain antibody may be an scFv.

In the present invention, the monoclonal antibody or the antigen-binding fragment thereof may specifically bind to PDL-1, wherein the PDL-1 may be derived from a canine or a human.

In another aspect, the present invention is directed to an isolated nucleic acid molecule including a nucleotide sequence encoding the heavy chain variable region (V_{H}) of the monoclonal antibody or the antigen-binding fragment thereof.

In the present invention, the isolated nucleic acid molecule may include a nucleotide sequence selected from the group consisting of SEQ ID NOs: 97, 99, 101, 103, 105, and 107.

In accordance with another aspect, provided is an isolated nucleic acid molecule including a nucleotide sequence encoding the light chain variable region (V_{L}) of the monoclonal antibody or an antigen-binding fragment thereof.

In the present invention, the isolated nucleic acid molecule may include a nucleotide sequence selected from the group consisting of SEQ ID NOs: 98, 100, 102, 104, 106, and 108.

In another aspect, the present invention is directed to a vector containing the isolated nucleic acid molecule.

In another aspect, the present invention is directed to a host cell containing the isolated nucleic acid molecule or the vector.

In another aspect, the present invention is directed to a pharmaceutical composition for treating a tumor containing the monoclonal antibody or the antigen-binding fragment thereof, and a pharmaceutically acceptable carrier or excipient.

In the present invention, the tumor may be osteosarcoma, melanoma, renal cancer, prostate cancer, bladder cancer, colorectal cancer, gastric cancer, liver cancer, non-small cell lung cancer, breast cancer, esophageal cancer, pancreatic cancer, glioma, ovarian cancer, or leukemia, but is not limited thereto.

In the present invention, the tumor may be a canine tumor or a human tumor.

In another aspect, the present invention is directed to a method of treating or preventing a tumor in a canine, including administering, to the canine having the tumor, the monoclonal antibody or the antigen-binding fragment thereof, or the pharmaceutical composition for treating a tumor.

In the present invention, the tumor may be osteosarcoma, melanoma, renal cancer, prostate cancer, bladder cancer, colorectal cancer, gastric cancer, liver cancer, non-small cell lung cancer, breast cancer, esophageal cancer, pancreatic cancer, glioma, ovarian cancer, or leukemia, but is not limited thereto.

In another aspect, the present invention is directed to a method of treating or preventing a tumor including administering the monoclonal antibody or the antigen-binding fragment thereof, or the pharmaceutical composition for treating a tumor.

In another aspect, the present invention is directed to a use of the monoclonal antibody or the antigen-binding fragment thereof, or the pharmaceutical composition for treating a tumor, for the treatment or prevention of a tumor.

In another aspect, the present invention is directed to a use of the monoclonal antibody or the antigen-binding fragment thereof, or the pharmaceutical composition for treating a tumor, for the preparation of a drug for treating or preventing a tumor.

As used herein, the term "PDL-1 protein" encompasses all such sequences, including the sequences described above and natural or artificial variants thereof. In addition, when a sequence fragment of the PDL-1 protein is referred to, it also refers not only to the described sequence fragment but also to the corresponding sequence fragments of natural or artificial variants thereof.

The term "antibody" refers to an immunoglobulin molecule typically composed of two pairs of polypeptide chains, each pair consisting of a light (L) chain and a heavy (H) chain. Antibody light chains may be classified as κ (kappa) or λ (lambda) chains. Heavy chains may be classified as µ, δ, γ, α, or ε, and the corresponding antibodies are defined as IgM, IgD, IgG, IgA, and IgE, respectively. In both the light and heavy chains, the variable region and the constant region are connected by a "J" region consisting of about 12 or more amino acids, and the heavy chain additionally includes a "D" region consisting of about 3 or more amino acids. Each heavy chain consists of a heavy chain variable region (VH) and a heavy chain constant region (CH). The heavy chain constant region consists of three domains (CH1, CH2, and CH3). Each light chain consists of a light chain variable region (VL) and a light chain constant region (CL). The light chain constant region consists of a single domain (CL). The constant region of an antibody is capable of mediating the binding of the immunoglobulin to host tissues or factors, such as various immune system cells (e.g., effector cells) and the first component (C1q) of the classical complement system. The VH and VL regions may be further divided into highly variable regions referred to as "complementarity-determining regions" (CDRs) and conserved regions referred to as "frameworks" (FRs) in which the CDRs are disposed. Each VH and VL region consists of three CDRs and four FRs disposed in the following order from the amino terminus to the carboxyl terminus: FR1, CDR1, FR2, CDR2, FR3, CDR3, and FR4. The variable regions of the heavy and light chains (VH and VL) form the antigen-binding site. The amino acid assignment of each domain is defined according to Kabat, Sequences of Proteins of Immunological Interest (National Institutes of Health, Bethesda, Md., 1987 and 1991), or according to the definitions of Chothia & Lesk (J. Mol. Biol. 196:901-917, 1987; Chothia et al., Nature 342:878-883, 1989).

The term "antibody" is not limited to any particular method of antibody production. For example, the antibody includes recombinant antibodies, monoclonal antibodies, and polyclonal antibodies. The antibody may be of different isotypes or subisotypes, for example, IgG (e.g., IgG1, IgG2, IgG3, or IgG4 subtype), IgA1, IgA2, IgD, IgE, or IgM antibodies.

As used herein, the term "antigen-binding fragment" of an antibody refers to a polypeptide including a portion of a full-length antibody, which retains the ability to specifically bind to the same antigen as the full-length antibody and/or the ability to compete with the full-length antibody for antigen-specific binding, which is known as the "antigen-binding part" of the antibody. As described in Fundamental Immunology, Ch. 7 (Paul, W., ed., second edition, Raven Press, N.Y., 1989), which is incorporated herein by reference in its entirety for all purposes. Antigen-binding fragments may be produced by recombinant DNA techniques or by enzymatic or chemical cleavage of a complete antibody. In some cases, antigen-binding fragments include Fab, Fab', F(ab')₂, Fd, Fv, dAb, and complementarity-determining region (CDR) fragments, single-chain antibody fragments (e.g., scFv), chimeric antibodies, diabodies, and polypeptides including at least a portion of a polypeptide sufficient to confer antigen-specific binding activity of an antibody.

As used herein, the term "Fd fragment" refers to an antibody fragment composed of VH and CH1 domains; the term "Fv fragment" refers to an antibody fragment composed of the VL and VH domains of an antibody; the term "dAb fragment" refers to an antibody fragment composed of a VH domain (Ward et al., Nature 341:544-546 (1989)); the term "Fab fragment" refers to an antibody fragment composed of VL, VH, CL, and CH1 domains; and the term "F(ab')₂ fragment" refers to an antibody fragment including two Fab fragments linked by a disulfide bond in the hinge region.

In some cases, the antigen-binding fragment of an antibody is a single-chain antibody (e.g., scFv) in which the VL and VH domains are linked by a linker
to form a monovalent molecule including a single polypeptide chain (e.g., Bird et al., Science 242:423-426 (1988); Huston et al., Proc. Natl. Acad. Sci. USA 85:5879-5883 (1988)). Such scFv molecules may have the general structure: NH₂-VL-Linker-VH-COOH or NH₂-VH-Linker-VL-COOH. Suitable linker sequences may include repeated GGGGS amino acid sequences or variants thereof. For example, (GGGGS)₄ may be used and variants thereof may also be used (Holliger et al., Proc. Natl. Acad. Sci. USA 90:6444-6448 (1993)).

Other linkers that may be used in the present invention are disclosed in Alfthan et al. (1995), Protein Eng. 8:725-731, Choi et al. (2001) Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol.

In some cases, the antigen-binding fragment of an antibody may be a diabody, that is, a bivalent antibody in which VH and VL are expressed on a single polypeptide chain, wherein a very short linker is used to prevent pairing of the two domains derived from the same chain, thereby forcing the domains to pair with complementary domains from another chain to form two antigen-binding sites. (e.g., Holliger P. et al., Proc. Natl. Acad. Sci. USA 90:6444-6448 (1993), and Poljak R. J. et al., Structure 2:1121-1123 (1994)).

In other cases, the antigen-binding fragment of an antibody may be a "bispecific antibody", defined as a first antibody (fragment) and a second antibody (fragment) or antibody mimetic coupled by a coupling arm. Non-limiting examples of coupling methods include chemical reactions, genetic fusion, and enzymatic reactions. The antigen-binding fragment may also be a "multispecific antibody" such as a trispecific antibody or a tetraspecific antibody, wherein the former is capable of specifically binding to three antigens and the latter is capable of specifically binding to four antigens. For example, a designed ankyrin repeat protein (DARPin) may be linked to or combined with an IgG antibody or an scFv-Fc antibody fragment, as disclosed in CN104341529A; and an anti-IL-17A fynomer may be linked to or combined with an anti-IL-6R antibody, as disclosed in WO2015141862A1.

In another case, the antigen-binding fragment of an antibody may be a "bispecific antibody conjugate" defined as a conjugate in which a first antibody (fragment) and a second biologically functional fragment (neither an antibody nor an antibody mimetic) are coupled via a coupling arm. Non-limiting examples of coupling methods include chemical reactions, genetic fusion, and enzymatic reactions. Examples of the second biologically functional fragment include peptides having binding activity, proteins, polyethylene glycol (PEG), radionuclides, nucleic acids, small-molecule toxins, receptors, or ligands. Since the conjugate retains the activity of each fragment, it exhibits dual functions or bispecificity.

The antigen-binding fragment of the present invention (e.g., the antibody fragment described above) may be obtained from the corresponding antibody (in the present invention, for example, 5C10, 5C10H1L1, 5C10H1L2, 5C10H2L1, and 5C10H2L2) by conventional techniques known to those skilled in the art, such as recombinant DNA techniques or enzymatic or chemical cleavage methods. The same specific screening methods may also be applied to the antigen-binding fragments as the intact antibody.

Unless explicitly mentioned otherwise, as used herein, the term "antibody" includes not only an intact antibody but also an antigen-binding fragment of the antibody.

As used herein, the terms "mAb" and "monoclonal antibody" refer to an antibody or an antibody-derived fragment from a highly homologous group, wherein the group consists of identical antibody molecules unless naturally occurring mutations are present. Monoclonal antibodies exhibit a high degree of specificity for a single epitope on an antigen. Polyclonal antibodies differ from monoclonal antibodies in that polyclonal antibodies typically include two or more different antibodies that recognize multiple epitopes on the same antigen. Monoclonal antibodies may generally be obtained using the hybridoma technique first described by Köhler et al. (Nature, 256:495, 1975) and may also be obtained using recombinant DNA techniques (U.S. Patent No. 4,816,567).

As used herein, the term "chimeric antibody" refers to an antibody including a portion of a light chain and/or heavy chain of an antibody (which may be derived from a particular species or belong to a particular antibody class or subclass) and another portion of a light chain and/or heavy chain of another antibody (which may be derived from the same or a different species or belong to the same or a different antibody class or subclass), while retaining binding activity to a target antigen (U.S. Patent No. 4,816,567, Cabilly et *al.;* Morrison et al., Proc. Natl. Acad. Sci. USA, 81:6851-6855 (1984)).

As used herein, the term "vector" refers to a nucleic acid vehicle into which a polynucleotide may be inserted. An expression vector is a vector capable of expressing a protein encoded by the inserted polynucleotide. A vector may be introduced into a host cell by transformation, transduction, or transfection, thereby enabling expression of a genetic element delivered to the host cell. Vectors are well known to those skilled in the art and non-limiting examples thereof include plasmids, phagemids, cosmids, artificial chromosomes (e.g., yeast artificial chromosomes (YAC), bacterial artificial chromosomes (BAC), or P1-derived artificial chromosomes (PAC)), bacteriophages (e.g., λ phage or M13 phage), and animal viruses. Non-limiting examples of animal viruses that may be used as vectors include retroviruses (e.g., lentiviruses), adenoviruses, adeno-associated viruses, herpesviruses (e.g., herpes simplex virus), poxviruses, baculoviruses, papillomaviruses, and papovaviruses (e.g., SV40). The vector may include various expression control elements and non-limiting examples thereof include promoter sequences, transcription initiation sequences, enhancer sequences, selectable markers, and reporter genes. In addition, the vector may include an origin of replication.

As used herein, the term "host cell" refers to a cell that may be used to introduce a vector and non-limiting examples thereof include: prokaryotic cells, such as *Escherichia coli* or *Bacillus subtilis;* fungal cells, such as yeast cells or *Aspergillus;* insect cells, such as Drosophila S2 cells or Sf9 cells; or animal cells, such as fibroblasts, CHO cells, COS cells, NS0 cells, HeLa cells, BHK cells, HEK293 cells, or human cells.

As used herein, the term "specific binding" refers to a non-random binding reaction between two molecules, for example, the interaction between an antibody and a target antigen thereof. In some embodiments, specific binding of an antibody to an antigen (or an antibody specific for an antigen) refers to an antibody having a binding affinity (KD) for the antigen of less than 10⁻⁵ M, for example less than 10⁻⁶ M, less than 10⁻⁷ M, less than 10⁻⁸ M, less than 10⁻⁹ M, less than 10⁻¹⁰ M, or even lower.

As used herein, the term "pharmaceutically acceptable vehicle and/or excipient" refers to a vehicle and/or excipient well known in the art that may be used in the pharmaceutical and physiological fields along with a subject to whom the composition is administered and an active ingredient (e.g., Remington's Pharmaceutical Sciences, Edited by Gennaro A.R., 19th ed., Pennsylvania: Mack Publishing Company, 1995). Non-limiting examples thereof include: pH-adjusting agents, surfactants, adjuvants, and ionic strength enhancers. For example, non-limiting examples of pH-adjusting agents: include phosphate-buffered saline (PBS); non-limiting examples of surfactants include: cationic, anionic, or non-ionic surfactants, such as Tween-80; and non-limiting examples of ionic strength enhancers include sodium chloride.

As used herein, the term "adjuvant" refers to a non-specific immune-stimulating substance that enhances an immune response to an antigen or alters the type of immune response when administered to a subject prior to or in combination with the antigen. Various types of adjuvants are known and non-limiting examples thereof include aluminum adjuvants (e.g., aluminum hydroxide), Freund's adjuvants (e.g., complete Freund's adjuvant and incomplete Freund's adjuvant), Corynebacterium parvum, lipopolysaccharide, and cytokines. Freund's adjuvant is one of the most commonly used adjuvants in animal experiments, whereas aluminum hydroxide adjuvants are more frequently used in clinical studies.

As used herein, the term "effective amount" refers to an amount sufficient to achieve, or at least partially achieve, a desired effect. For example, an effective amount for preventing a disease such as a tumor refers to an amount sufficient to prevent, inhibit, or delay the onset of the disease, such as a tumor. An effective amount for treating a disease refers to an amount sufficient to cure or at least inhibit the disease and complications thereof in a subject suffering from the disease. Such an effective amount is determined by those skilled in the art. For example, an effective amount for treating a disease may be determined depending on factors such as the severity of the disease, the overall immune system condition of the patient, and the general condition of the patient, including age, body weight, and gender, as well as drug delivery methods and other treatments administered simultaneously.

### [Example]

Hereinafter, the present invention will be described in more detail with reference to examples. However, it will be obvious to those skilled in the art that these examples are provided only for illustration of the present invention and should not be construed as limiting the scope of the present invention.

The cell lines and experimental methods used in the following Examples are described below.

### Sources of cell lines used and cell infection method

The canine macrophage cell line DH82 and the canine osteosarcoma cell line D17 used in Example of the present invention were purchased from ATCC (Manassas, VA, USA). The cells were cultured in DMEM containing 4.5 g/L glucose, L-glutamine, and sodium pyruvate (Corning, USA), supplemented with 10% v/v fetal bovine serum (FBS) (Gibco, USA) and 1% v/v antibiotic-antimycotic solution (Welgene, Korea). HEK-293F cells were cultured in FreeStyle^{™}293 expression medium (Gibco, USA). FreeStyle^{™} 293-F cells (Gibco, USA) were transfected using FectoPRO transfection reagent (Polyplus, Illkirch-Graffenstaden, France).

### Phage Display Panning

In order to screen phage display panning antibodies, canine antibody clones that bind to canine PD-L1 were obtained using a synthetic antibody library (provided by Prof. Hyun-Bo Shim, Ewha Womans University). This antibody library has a diversity of 7.6 × 10⁹ and the scFv was tagged with HA at the C-terminus. The scFv-displaying phages for each panning round were produced using ER2738 cells (NEB, New England Biolabs, USA) and VCSM13 helper phages, and library amplification and phage display panning were performed in accordance with the protocol disclosed in Phage Display: A Laboratory Manual (ISBN 978-087969740-2).

Specifically, the frozen library stock was thawed to obtain scFv-displaying phages, inoculated into Luria-Bertani (LB) medium (BD Biosciences, USA), and then subjected to the panning process by plating onto LB Agar Miller plates (BD Biosciences, USA) containing 1% D-glucose (Duchefa Biochemie, Netherlands) and 100 µg/ml carbenicillin (GoldBio, USA). After overnight incubation, cells containing phage DNA were inoculated into SB medium containing 1% w/v MOPS (Thermo Fisher Scientific, USA), 2% w/v yeast extract (Thermo Fisher Scientific), and 3% tryptone (Thermo Fisher Scientific), and incubated at 37°C for 2 hours. After incubation, the cells were transferred to fresh SB medium and approximately 10¹² CFU of VCSM13 helper phage (Stratagene, San Diego, CA, USA) were added to the cultured cells.

The cells were incubated with shaking at 37°C and 210 rpm for 2 hours and then an equal volume of fresh medium was added to the entire culture (final concentrations: 100 µg/ml of carbenicillin and 70 µg/ml of kanamycin). After overnight incubation, scFv-displaying phages were harvested using PEG precipitation and PD-L1-binding antibody phages were subjected to panning using ImmunoTubes (Thermo Fisher Scientific) coated with canine-derived PD-L1 antigen protein.

To reduce non-specific binding of scFv antibodies, ImmunoTubes coated with antigen were blocked using a blocking solution containing 0.1% PBS-T (PBS containing 0.1% Tween-20; iNtRON Biotechnology, Korea) and 5% w/v skim milk (BD Biosciences) or 5% w/v bovine serum albumin (BSA; HanLAB, Korea). The scFv phage samples were mixed with an antigen protein at room temperature for 2 hours. In order to obtain phages with high affinity, the ImmunoTubes that interacted with phages were washed with 0.1% PBS-T and the number of washing steps was increased as the panning rounds progressed. After washing, phages bound to canine PD-L1 were eluted using 0.25% trypsin solution (Gibco). The eluted phages were then reinfected into ER2738 cells for amplification and used for the next round of panning. A total of four rounds of panning were performed to select PD-L1-bound scFv phages. PD-L1 antigen binding of the selected scFv antibodies of phage-infected *E. coli* colonies obtained from the third and fourth panning rounds were verified using ELISA.

### scFv expression and TES periplasmic extraction

Single colonies were randomly selected from the *E. coli* colonies obtained after the third and fourth rounds of panning and incubated overnight in 96-deep well plates containing 1 mL/well of LB medium supplemented with 100 µg/mL carbenicillin. On the following day, an aliquot of the culture was diluted, inoculated into fresh LB medium and cultured with shaking until the cell density reached approximately OD600 ≈ 0.6. IPTG (Duchefa Biochemie) was then added to a final concentration of 1 mM, followed by overnight incubation to induce expression of the scFv protein. The scFv proteins expressed in the periplasmic space were extracted by osmotic shock using TES buffer (20% w/v sucrose (Duchefa Biochemie), 50 mM Tris-HCl (iNtRON), and 1 mM EDTA, pH 8.0 (BIONEER, Korea)) (DOI: 10.1186/s13568-020-01063-x). The scFv proteins expressed in the periplasmic space were obtained by osmotic shock extraction using TES buffer, were mixed with PD-L1 antigen protein and were then mixed with an anti-HA peroxidase-conjugated secondary antibody against the HA-tag fused to the C-terminus of the scFv (Sigma-Aldrich, #12013819001). The resulting mixture was used in an enzyme-linked immunosorbent assay (ELISA) to evaluate the PD-L1 binding activity of the individual antibodies. After confirming binding to the PD-L1 antigen protein, the scFv samples were incubated at 70°C for 10 minutes to determine the thermal stability of the scFv antibodies, followed by incubation on ice for 30 minutes. Then, ELISA was performed again to determine binding to PD-L1 antigen protein.

### Expression of IgG-format antibodies using two-vector system and purification of IgG antibodies

Sixteen unique antibodies having identified nucleotide sequences were amplified by PCR using primer sets capable of binding to the individual VH and VL domains and a KAPA HiFi HotStart PCR kit (Roche Sequencing, Indianapolis, USA). The VH and VL PCR products of each clone were digested with BamHI/NheI and BamHI/BsiWI restriction enzymes, respectively, and ligated into the corresponding restriction sites of the pCEP-VH and pCEP-VL vectors (doi: 10.3390/v12060684), which had been digested with the same restriction enzymes, using T4 DNA ligase (NEB) to obtain heavy-chain and light-chain expression vectors enabling expression of the 16 unique antibodies in IgG format in mammalian cells. The VH fragments were fused to the CH1-hinge-CH2-CH3 region for heavy-chain expression and the VL fragments were fused to the Cκ region for light-chain expression.

The resulting heavy-chain and light-chain expression vectors were co-transfected into FreeStyle^{™} 293-F cells using FectoPRO transfection reagent at a DNA ratio of 2:1 (light chain:heavy chain). After 9 days, the culture supernatant was harvested and IgG-format antibodies were purified by column chromatography using Protein A agarose beads (Repligen, USA). After purification and dialysis in DPBS at pH 7.5, antibody concentrations were quantified using a spectrophotometer, and the purification level and purity of the antibodies were evaluated by SDS-polyacrylamide gel electrophoresis (PAGE) and then Coomassie Brilliant Blue staining (Biosesang, Korea).

### Analysis of binding and inhibitory activities of scFv and IgG by ELISA

The binding and inhibitory activities of individual scFv and IgG antibodies were analyzed by ELISA. For the binding assay, 96-well ELISA plates were coated with canine PD-L1-hFc protein at a concentration of 500 ng/mL in PBS and incubated overnight at 4°C. The solution in the wells was discarded and the wells were blocked with 3% (w/v) skim milk in 0.1% PBS-T by incubation at 37 °C for 1 hour. Subsequently, the wells were treated with 100 µL/well of periplasmic extracts containing scFv or 200 ng/well of purified IgG and incubated at room temperature for 2 hours. The plates were washed with tap water, followed by incubation with horseradish peroxidase (HRP)-conjugated anti-HA antibody (1:5,000; Roche, Basel, Switzerland) in blocking buffer at 37°C for 45 minutes.

For the inhibition assay, the ELISA plates were coated with canine PD-L1 under the same conditions described above. The ELISA plates were treated with periplasmic extracts containing scFv (100 µL/well) mixed with 100 ng/well of human Cκ-tagged human PD-1, incubated at room temperature for 2 hours, and washed with tap water. Then, HRP-conjugated anti-human Cκ antibody (Jackson ImmunoResearch, Pennsylvania, USA) diluted in blocking buffer was added, followed by incubation under the same conditions. For detection, 50 µL/well of 3,3',5,5'-tetramethylbenzidine (TMB; BD Biosciences) was added and absorbance was measured at 450 nm using a BioTek Epoch microplate reader (Agilent, California, USA).

### Flow Cytometry Analysis

BD FACSVerse^{™} (BD Biosciences) was used to analyze specific binding of purified IgG to canine PD-L1 expressed on the cell surface. 1 × 10⁵ DH82 and D17 cells were used in each experiment. The cells were resuspended in enzyme-free PBS-based cell dissociation buffer (Gibco). The cells were washed with FACS buffer containing TBS (iNtRON), 0.5% BSA (HanLAB), and 0.05% NaN₃ (Acros Organics), and then resuspended in FACS buffer containing purified IgG (25 µg/mL) and incubated at 4°C for 1 hour. After washing with FACS buffer, each IgG clone was treated with Alexa Fluor 488-conjugated anti-human Fc antibody (1:500; Jackson ImmunoResearch, West Grove, PA, USA) or Alexa Fluor 647-conjugated anti-human Fc antibody (1:500; Jackson ImmunoResearch) and incubated at 4°C for 1 hour. Finally, all samples were measured using a FACSVerse^{™} flow cytometer and analyzed using FlowJo software (FlowJo LLC, Oregon, USA).

### Example 1: Identification of scFv antibodies specifically binding to canine PD-L1

Single-chain variable fragments (scFvs) binding to canine PD-L1 were screened using phage display technology. A synthetic scFv library with a diversity of 7.6 × 10⁹ was used (FIG. 1A). The diversity of the selected scFv phage candidates was evaluated by testing the scFv phages obtained through panning. The infection titer measured after the fourth round of panning using ER2738 cells (New England Biolabs) increased to 3.5 × 10⁹ CFU compared with the titer obtained after the third round of panning (4.3 × 10⁷ CFU), which indicates that scFv antibodies binding to canine PD-L1 were increased in the panning pool (FIG. 1B).

The binding of respective scFv clones selected from the clones obtained through the panning process to canine PD-L1 was identified by ELISA. Thirty-five scFv clones having relatively high binding activity (OD value ≥ 0.2) to canine PD-L1 were obtained and indicated by red arrows (FIG. 1C). The thermal stability of the 35 selected clones was subsequently evaluated. For this evaluation, the scFv clones were heat-treated at 70°C to induce structural changes, followed by cooling to allow structural refolding.

ELISA results showed that binding to canine PD-L1 was maintained even after heat treatment, demonstrating the thermal stability of the scFv clones (FIG. 1D).

The DNA sequences of the scFvs of all 35 clones were analyzed. As a result of the analysis, different sequences in the CDR regions were identified for 19 scFv clones.

As a result, a diverse scFv library using phage display technology was screened to produce 19 unique scFv clones with high affinity and thermal stability that specifically target canine PD-L1.

### Example 2: Screening of scFvs for inhibition of canine PD-L1 binding to human PD-1

In this example, an assay system was established to evaluate the inhibitory activity of canine PD-L1 neutralizing antibody clones.

The amino acid sequence homology between canine PD-L1 and human PD-L1 was 76.04%. However, identical amino acid sequences were observed in several regions, as highlighted in purple in FIG. 2A. In particular, the regions indicated by red boxes represent the critical binding sites for human PD-1, suggesting that the binding sites of canine and human PD-L1 are similar (FIG. 2A). Accordingly, ELISA was performed to verify the binding of the candidate scFvs to human PD-L1 and the results showed that specific scFvs were capable of binding to human PD-L1 (FIG. 2B).

In the present invention, the functions of the scFvs were evaluated based on the assumption that the desired antibodies should bind to PD-L1 in a manner similar to that of human PD-1. In this example, the potential of HA-tagged canine PD-L1 scFvs to interfere with the interaction between human Fc-fused canine PD-L1 and human Cκ-fused human PD-1 was evaluated. The inhibitory effect of the scFvs on the interaction between canine PD-L1 and human PD-1 was quantified using an HRP-conjugated anti-human Cκ secondary antibody that recognizes human PD-1 (FIG. 2C).

During analysis of the competitive ELISA results, a decrease in OD450 was observed when scFvs were introduced, compared with samples treated only with canine PD-L1 and human PD-1 proteins. These results strongly suggest that the scFvs effectively inhibit the interaction between canine PD-L1 and human PD-1 (FIG. 2D). Based on the results of evaluation of binding affinity to canine PD-L1, thermal stability, and PD-1/PD-L1 blocking activity, clones having superior binding affinity to canine PD-L1 were prioritized and then converted into the IgG format.

### Example 3: Functional validation of IgG antibodies derived from canine PD-L1-specific scFvs

Based on the results obtained in Examples 1 and 2, six scFvs with the highest priority (#1-#6) were selected and cloned for conversion into IgG format.

An scFv includes the variable regions of an immunoglobulin heavy chain (VH) and light chain (VL) bound by a linker. The VH and VL domains were cloned and inserted into expression vectors capable of forming human IgG using a two-vector system (FIG. 3A). It was confirmed that the expressed IgG antibodies were properly assembled through disulfide bonds to form a structure consisting of two heavy chains and two light chains. This was verified by SDS-PAGE under reducing and non-reducing conditions. Protein bands observed in all six samples indicate the presence of intact IgG molecules (FIG. 3B).

Since the IgG-format antibodies converted from scFvs might have lost the binding activity thereof to canine PD-L1, *in vitro* binding assays were performed using these IgG antibodies. The binding activities of the antibodies to both canine PD-L1 and human PD-L1 were evaluated. An anti-human PD-L1 antibody capable of binding to both human and canine PD-L1, KL-001 (KR102357951B1), and atezolizumab (Genentech, US8217149) were used as positive controls. Measurement of OD at 450 nm showed that the binding affinity to canine PD-L1 was consistently maintained (FIG. 3C).

In addition, cell-based *in vitro* binding assays were performed using canine cell lines expressing canine PD-L1, specifically DH82 macrophages and D17 canine osteosarcoma cells, followed by flow cytometry. To evaluate binding by detecting fluorescence signals, the six IgG antibodies were incubated with DH82 and D17 cells. In this analysis, KL-001 was used as a positive control.

As a result, strong binding signals were observed for all six samples in the DH82 cell line, whereas binding signals were observed in all clones excluding #3 and #5 in the D17 cell line (FIGs. 3D and 3E).

Accordingly, the results of the present example showed that the IgG antibodies derived from the scFvs exhibited significant functional binding to canine PD-L1. Based on binding affinity and sequence specificity, two antibodies, #1 and #6, were selected as candidate antibodies for further analysis.

### Example 4: Selection of αPD-L1 antibody candidates having antitumor effects against canine osteosarcoma

In a previous study by the present inventors, novel αPD-L1 antibody candidates capable of binding to both canine and human PD-L1 were developed. After confirming the affinity of these antibodies for canine PD-L1 *in vitro,* further studies were conducted to evaluate the effects thereof *in vivo.* A canine osteosarcoma mouse model was established by xenografting D17 cells (4 × 10⁶ cells/mouse), which is a canine osteosarcoma cell line, into NOG mice (KOATECH, Korea), which are deficient in T cells, B cells, and NK cells.

Ten days after tumor xenografting in the mouse model, in order to evaluate the antitumor efficacy of the antibodies, antibody #1 or #6 was intravenously administered to NOG mice having D17 tumors that had administered 8 × 10⁶ canine PBMCs. Mice administered PBMCs treated with antibody #1 or #6 had reduced tumor size compared with the control group receiving PBMCs alone (FIG. 4A). In particular, mice administered PBMCs treated with antibody #1 had the smallest tumor volumes among the experimental groups. In addition, the result of measurement of tumor weight on the day of sacrifice showed that the group administering PBMCs treated with antibody #1 had the least tumor growth compared with the other groups (FIG. 4B). These results demonstrate that antibodies #1 and #6 bind to canine PD-L1 and exert antitumor effects.

### [Sequence Listing Free Text]

An electronic file is attached.

## Claims

1. A monoclonal antibody or an antigen-binding fragment thereof,
wherein the monoclonal antibody comprises:
a heavy chain variable region (V_{H}) comprising HCDR1 comprising a sequence of SEQ ID NO: 14, HCDR2 comprising a sequence of SEQ ID NO: 16, and HCDR3 comprising a sequence of SEQ ID NO: 18; and
a light chain variable region (V_{L}) comprising LCDR1 comprising a sequence of SEQ ID NO: 21, LCDR2 comprising a sequence of SEQ ID NO: 23, and LCDR3 comprising a sequence of SEQ ID NO: 25.

2. The monoclonal antibody or an antigen-binding fragment thereof according to claim 1, wherein an amino acid sequence of the heavy chain variable region (V_{H}) of the monoclonal antibody or the antigen-binding fragment thereof comprising a sequence of SEQ ID NO: 1, and
an amino acid sequence of the light chain variable region (V_{L}) of the monoclonal antibody or the antigen-binding fragment thereof comprising a sequence of SEQ ID NO: 2.

3. The monoclonal antibody or an antigen-binding fragment thereof according to claim 1, wherein the monoclonal antibody or the antigen-binding fragment thereof is selected from Fab, Fab', F(ab')₂, Fd, Fv, dAb, a single-chain antibody, a humanized antibody, a chimeric antibody, and a diabody.

4. The monoclonal antibody or an antigen-binding fragment thereof according to claim 1, wherein the single-chain antibody is an scFv.

5. The monoclonal antibody or an antigen-binding fragment thereof according to claim 1, wherein the monoclonal antibody or the antigen-binding fragment thereof specifically binds to PDL-1.

6. The monoclonal antibody or an antigen-binding fragment thereof according to claim 5, wherein the PDL-1 is derived from a canine or a human.

7. A monoclonal antibody or an antigen-binding fragment thereof,
wherein the monoclonal antibody comprises:
a heavy chain variable region (V_{H}) comprising:
HCDR1 comprising a sequence of SEQ ID NO: 28, SEQ ID NO: 42, SEQ ID NO: 56, SEQ ID NO: 70, or SEQ ID NO: 84;
HCDR2 comprising a sequence of SEQ ID NO: 30, SEQ ID NO: 44, SEQ ID NO: 58, SEQ ID NO: 72, or SEQ ID NO: 86; and
HCDR3 comprising a sequence of SEQ ID NO: 32, SEQ ID NO: 46, SEQ ID NO: 60, SEQ ID NO: 74, or SEQ ID NO: 88; and
a light chain variable region (V_{L}) comprising:
LCDR1 comprising a sequence of SEQ ID NO: 35, SEQ ID NO: 49, SEQ ID NO: 63, SEQ ID NO: 77, or SEQ ID NO: 91;
LCDR2 comprising a sequence of SEQ ID NO: 37, SEQ ID NO: 51, SEQ ID NO: 65, SEQ ID NO: 79, or SEQ ID NO: 93; and
LCDR3 comprising a sequence of SEQ ID NO: 39, SEQ ID NO: 53, SEQ ID NO: 67, SEQ ID NO: 81, or SEQ ID NO: 95.

8. The monoclonal antibody or an antigen-binding fragment thereof according to claim 7, wherein the monoclonal antibody or the antigen-binding fragment thereof is selected from the group consisting of:
(i) a monoclonal antibody or an antigen-binding fragment thereof comprising:
a heavy chain variable region (V_{H}) comprising an amino acid sequence of SEQ ID NO: 3; and
a light chain variable region (V_{L}) comprising an amino acid sequence of SEQ ID NO: 4;
(ii) a monoclonal antibody or an antigen-binding fragment thereof comprising:
a heavy chain variable region (V_{H}) comprising an amino acid sequence of SEQ ID NO: 5; and
a light chain variable region (V_{L}) comprising an amino acid sequence of SEQ ID NO: 6;
(iii) a monoclonal antibody or an antigen-binding fragment thereof comprising:
a heavy chain variable region (V_{H}) comprising an amino acid sequence of SEQ ID NO: 7; and
a light chain variable region (V_{L}) comprising an amino acid sequence of SEQ ID NO: 8;
(iv) a monoclonal antibody or an antigen-binding fragment thereof comprising:
a heavy chain variable region (V_{H}) comprising an amino acid sequence of SEQ ID NO: 9; and
a light chain variable region (V_{L}) comprising an amino acid sequence of SEQ ID NO: 10; and
(v) a monoclonal antibody or an antigen-binding fragment thereof comprising:
a heavy chain variable region (V_{H}) comprising an amino acid sequence of SEQ ID NO: 11; and
a light chain variable region (V_{L}) comprising an amino acid sequence of SEQ ID NO: 12.

9. The monoclonal antibody or an antigen-binding fragment thereof according to claim 7, wherein the monoclonal antibody or the antigen-binding fragment thereof is selected from Fab, Fab', F(ab')₂, Fd, Fv, dAb, a single-chain antibody, a humanized antibody, a chimeric antibody, and a diabody.

10. The monoclonal antibody or an antigen-binding fragment thereof according to claim 7, wherein the single-chain antibody is an scFv.

11. The monoclonal antibody or an antigen-binding fragment thereof according to claim 7, wherein the monoclonal antibody or the antigen-binding fragment thereof specifically binds to PDL-1.

12. The monoclonal antibody or an antigen-binding fragment thereof according to claim 11, wherein the PDL-1 is derived from a canine or a human.

13. An isolated nucleic acid molecule comprising a nucleotide sequence encoding a heavy chain variable region (V_{H}) of the monoclonal antibody or antigen-binding fragment thereof according to claim 1.

14. The isolated nucleic acid molecule according to claim 13, wherein the nucleic acid molecule comprises a nucleotide sequence of SEQ ID NO: 97.

15. An isolated nucleic acid molecule comprising a nucleotide sequence encoding a light chain variable region (V_{L}) of the monoclonal antibody or antigen-binding fragment thereof according to claim 1.

16. The isolated nucleic acid molecule according to claim 15, wherein the nucleic acid molecule comprises a nucleotide sequence of SEQ ID NO: 98.

17. An isolated nucleic acid molecule comprising a nucleotide sequence encoding a heavy chain variable region (V_{H}) of the monoclonal antibody or antigen-binding fragment thereof according to claim 7.

18. The isolated nucleic acid molecule according to claim 17, wherein the nucleic acid molecule comprises a nucleotide sequence selected from the group consisting of SEQ ID NOs: 99, 101, 103, 105, and 107.

19. An isolated nucleic acid molecule comprising a nucleotide sequence encoding a light chain variable region (V_{L}) of the monoclonal antibody or antigen-binding fragment thereof according to claim 7.

20. The isolated nucleic acid molecule according to claim 19, wherein the isolated nucleic acid molecule comprises a nucleotide sequence selected from the group consisting of SEQ ID NOs: 100, 102, 104, 106, and 108.

21. A vector comprising the isolated nucleic acid molecule according to any one of claims 13 to 20.

22. A host cell comprising the isolated nucleic acid molecule according to any one of claims 13 to 20 or the vector according to claim 21.

23. A pharmaceutical composition for treating a tumor comprising:
the monoclonal antibody or antigen-binding fragment thereof according to any one of claims 1 to 12; and
a pharmaceutically acceptable carrier or excipient.

24. The pharmaceutical composition according to claim 23, wherein the tumor is osteosarcoma, melanoma, renal cancer, prostate cancer, bladder cancer, colorectal cancer, gastric cancer, liver cancer, non-small cell lung cancer, breast cancer, esophageal cancer, pancreatic cancer, glioma, ovarian cancer, or leukemia.

25. The pharmaceutical composition according to claim 23, wherein the tumor is derived from a canine or a human.

26. A method of treating or preventing a tumor in a canine or an animal, comprising administering, to the canine or animal having the tumor, the monoclonal antibody or the antigen-binding fragment thereof according to any one of claims 1 to 12, or the pharmaceutical composition for treating a tumor according to claims 23.

27. The method according to claim 26, wherein the tumor is selected from the group consisting of osteosarcoma, melanoma, renal cancer, prostate cancer, bladder cancer, colorectal cancer, gastric cancer, liver cancer, non-small cell lung cancer, breast cancer, esophageal cancer, pancreatic cancer, glioma, ovarian cancer, and leukemia.
